# EUROPEAN PATENT SPECIFICATION

(11) **EP 1 799 195 B1**
(45) Date of publication and mention of the grant of the patent: **23.03.2016**
(21) Application number: 05769055.4
(22) Date of filing: 08.08.2005
(51) Int. Cl.: A61K 9/16, A61K 9/20, A61K 31/167, A61K 31/192

(54) **GRANULES COMPRISING PARACETAMOL, AN NSAID AND A SUGAR ALCOHOL MADE BY MELT EXTRUSION**
GRANULATE MIT PARACETAMOL, EINEM NSAID UND EINEM ZUCKERALKOHOL HERGESTELLT DURCH SCHMELZEXTRUSION
GRANULES COMPOSÉS DE PARACETAMOL, D'UN AINS ET D'UN POLYOL FABRIQUÉS PAR EXTRUSION DE MATIERE FONDUE

(30) Priority: 12.08.2004 GB 0417939; 12.08.2004 GB 0417942
(43) Date of publication of application: 27.06.2007
(73) Proprietor: Reckitt Benckiser Healthcare (UK) Limited, Slough, Berkshire SL1 3UH (GB)
(72) Inventor: SHERRY, Robert, Nottingham NG2 3AA (GB)
(74) Representative: O'Brien, Niall James
(86) International application number: PCT/GB2005/003079
(87) International publication number: WO 2006/016126

(56) References cited:
- EP-A- 0 435 450
- WO-A-02/098387
- DE-A1- 19 809 242
- FR-A- 2 705 677
- US-A1- 2004 013 735
- NDINDAYINO F ET AL: "Direct compression properties of melt-extruded isomalt" INTERNATIONAL JOURNAL OF PHARMACEUTICS (KIDLINGTON), vol. 235, no. 1-2, 20 March 2002 (2002-03-20), pages 149-157, XP002349432 ISSN: 0378-5173

## Description

The present invention relates to compositions containing a non-steroidal anti-inflammatory drug, to processes to prepare them and uses thereof.

Non-steroidal anti-inflammatory drugs (NSAIDS) are a widely used class of medicaments. They are a well defined group of compounds and include phenylpropionic acids such as ibuprofen, naproxen, ketoprofen and flurbiprofen. They are primarily used for the treatment of one or more of pain, inflammation and fever, for example rheumatoid arthritis, ankylosing spondylitis, osteoarthritis, post-operative pain, post-partum pain and soft tissue injuries.

NSAIDS are generally acidic and substantially insoluble drugs. They are conveniently administered as an oral pharmaceutical composition in the form of tablets. Thus pharmaceutically acceptable excipients must be chosen for combination with the NSAID, with which the NSAID is compatible and with which it can form tablets having a satisfactory hardness and also release the medicament rapidly into the body so that it is available for absorption.

A major issue in connection with the disorders identified above is to improve the onset of action of the NSAID, particularly in the treatment of pain. It is believed that rapid disintegration of a formulation releases the drug into the body quickly leading to a more rapid onset of therapeutic action compared with a standard dosage form. Accordingly, it is desired to produce a solid dosage form for oral administration adapted to disintegrate quickly in the gastro-intestinal tract. However, as many of the NSAIDS are acidic drugs, accordingly, absorption can be a problem in the acidic conditions encountered in the stomach. Furthermore, although the literature has proposed many formulations adapted to disintegrate quickly, a major problem occurs with ibuprofen and other NSAIDS as they may need to be administered in relatively high doses, e.g. up to 800 mg per unit dose. Thus, there is a problem to provide a dosage form which includes the NSAID together with excipients useful to formulate the tablet into the dosage form and also excipients useful to ensure rapid disintegration, but not to provide a tablet that is too large for patient consumption or cannot be produced according to standard large scale manufacturing processes. Furthermore, the solid dosage form must be sufficiently hard to withstand the rigours of the manufacturing process (for example as encountered during the stage of film coating in a perforated rotating drum and packaging etc) but must have appropriate disintegration characteristics to ensure rapid release of the drug from the formulation and also appropriate dissolution characteristics. Another significant problem that must be overcome is to ensure that the composition is capable of being compressed with standard tabletting machinery without sticking to the punches of the tabletting machine.

In this respect, WO 01/41733 by The Boots Company PLC discloses that if a disintegrating agent is incorporated into a molten NSAID and intimately combined therewith and then is cooled and milled to produce a granule, a composition capable of tabletting with minimum tabletting excipients and having advantageous tabletting, disintegration and dissolution properties is provided, if silicon dioxide is incorporated therein.

A further alternative approach to increase the bioavailability of an NSAID is to administer the NSAID in the form of a salt, as such salts are typically more soluble than the corresponding free acid. In this respect, German Patent Application 3922441 A seeks to improve the tablettability of ibuprofen compositions and discloses that this may be achieved by converting ibuprofen wholly or partially into its calcium salt and using these for tabletting. It is said that the compositions may optionally contain ibuprofen, S(+)-ibuprofen or their ammonium, sodium or potassium salts. The calcium salt and the optional other ibuprofen actives may be incorporated into the tablet as separately produced compounds or the salts may be formed in-situ during the tablet preparation method through the reaction between ibuprofen (an acidic drug) with a solution or suspension of a reactant comprising one or more of CaO, Ca(OH)₂, CaCO₃, NaOH, KOH, NH₄OH, Na₂CO₃, NaHCO₃, K₂CO₃, KHCO₃, (NH₄)₂CO₃, NH₄HCO₃ (in an amount of 25% to 110% of the equivalent quantity of ibuprofen). The mixture obtained is then granulated, dried if appropriate, and then tabletted after the optional incorporation of other excipients. The specification comments that depending on the proportions of other salts used with the calcium salt, the ammonium and alkali salts improve the solubility of the calcium salt-containing compositions and thus control the bioavailability, but they also increase the hygroscopicity and stickiness.

This is a particular problem associated with processing NSAIDS in the form of a salt as these materials are typically poorly compressible. Suitably, NSAIDS in the form of salts in comparison to the free acid form are flaky, soft and sticky materials and they do not lend themselves to formulation into a dosage form as they are particularly difficult to compress in comparison to the corresponding free acid. Consequently, NSAIDS in the form of salts may stick to the punches of the tabletting machine. Furthermore, NSAIDS in the form of salts are typically difficult to pre-granulate prior to compression with other excipients into tablets. It is thus usually necessary to subject the NSAID salt to an initial treatment stage, such as a granulation process, in order to form satisfactory tablets. In particular, the ammonium and alkali metal salts of NSAIDS, such as the propionic acid derivatives i.e. ibuprofen, are known as sticky, hygroscopic and poorly compressible substances. The sodium salt of ibuprofen, due to its waxy nature, is regarded as exceptionally poorly compressible and also as having a poor ability to be granulated. This is one of the main reasons why very few sodium ibuprofen containing tablets are presently available.

Still further it may be desirable to provide a pharmaceutical composition which includes a further analgesic active agent in addition to the NSAID i.e. a combination product. One such analgesic agent which has been combined with NSAIDS is acetaminophen (also known as paracetamol) which displays analgesic (fever relief) and antipyretic (fever relief) properties, but essentially no anti-inflammatory action. Such products are, amongst other things, extremely effective for treating pain and fever i.e. headache, muscle and joint pain, back ache, cough, cold and flu. Typically, the NSAID in such a combination product is in the form of the free acid as the problems associated with using NSAIDS salts for oral administration, as discussed above, apply equally. In addition, there are further problems associated with providing an oral dosage form (i.e. tablet) containing paracetamol.

In particular, paracetamol is a fine soft powder, and as such, it does not lend itself for compressing directly into tablets. Thus, it is necessary to pre-treat the paracetamol prior to compression into a solid dosage form. Typically, the paracetamol is subjected to a wet granulation process to produce a granular form of paracetamol which is subsequently mixed with additional tabletting excipients and the resultant mixture compressed to form tablets: Alternatively, direct compression forms of paracetamol, which have typically been subjected to a pre-treatment process, may be obtained from specialist suppliers. These pre-treatment requirements typically increase the complexity and cost associated with processes for producing solid dosage forms of paracetamol. Moreover, paracetamol is typically administered in relatively high doses e.g. up to 500 mg per unit dose. Thus, there is also a problem to provide a dosage form which includes the paracetamol together with excipients useful to formulate the tablet into the dosage form and also excipients useful to ensure rapid disintegration, but not to provide a tablet that is too large for patient consumption or cannot be produced according to standard large scale manufacturing process. Suitably, this problem is typically magnified when paracetamol is combined with another pharmaceutically active agent (i.e. an NSAID) which may also be administered in a relatively high dose.

Ndindayina et al., Int. J. Pharm, 235 (2002), 149 - 157, describe paracetamol tablets comprising melt-extruded isomalt.

We have now found that if a mixture comprising a molten sugar alcohol having an NSAID in the form of a salt (referred to as an NSAID salt) and paracetamol contained therein is solidified and formed into granules, a composition capable of tabletting with minimum tabletting excipients and having advantageous tabletting, disintegration and dissolution properties is provided.

Thus according to a first aspect the present invention provides a pharmaceutical composition comprising a granular component comprising a plurality of solidified melt granules of a sugar alcohol having a salt of a non-steroidal anti-inflammatory drug (NSAID salt) and paracetamol contained therein.

Unexpectedly, the pharmaceutical composition typically exhibits improved flow characteristics and is less flaky/sticky than the NSAID salt itself.. Conveniently, the granular composition lends itself to formulation into solid dosage forms as it is easier to compress and tends not to stick to the punches of a tabletting machine. Moreover, the throughput of the compression process is substantially increased compared to employing untreated paracetamol or/and the NSAID salt itself. Furthermore, it is typically not necessary to pre-treat the NSAID salt and the paracetamol (i.e. employing a granulation process to improve its flowability) before forming a pharmaceutical composition according to the present invention. Suitably, NSAID salts and paracetamol used to form the pharmaceutical composition according to the present invention may be taken directly, without pre-treatment, from a bulk production process.

Further advantages of the pharmaceutical composition lie in the relatively small amount of additional tabletting excipients needed to prepare a dosage form, in particular a solid dosage form for oral administration, thus allowing smaller dosage forms to be produced having a relatively high concentration of NSAID and paracetamol thereby increasing patient compliance.

Unexpectedly, it has been found that pharmaceutical formulations prepared from the pharmaceutical composition of the present invention have valuable disintegrating properties. Moreover, the dissolution results of such formulations typically exhibit an unexpectedly high level of the NSAID and paracetamol dissolved in the aqueous medium after relatively short periods of time.

Thus the pharmaceutical composition of the present invention typically provides advantages in processing NSAID salts and paracetamol, improved patient compliance, improved disintegration and dissolution properties, and a lowering of the overall costs of tablets formed from NSAID salts and paracetamol.

In the preparation of the pharmaceutical composition the sugar alcohol is melted. Thus the terms "melt" and "molten" mean that the sugar alcohol must melt at least in part during formation of the granular composition. Preferably, the sugar alcohol is fully melted during the preparation of the pharmaceutical. composition.

Suitably, when the sugar alcohol is melted a liquid is formed. The NSAID salt may partially dissolve within the molten sugar alcohol; however the majority of the NSAID salt is typically dispersed within the molten sugar alcohol. Typically, as described hereinafter, the NSAID salt has a melting point far higher than the melting point of the sugar alcohol and the NSAID salt does not melt during formation of the pharmaceutical composition of the present invention. Paracetamol has a melting point of 169 to 172 °C and depending on the specific sugar alcohol employed and the process operating conditions, the paracetamol may partially or fully melt during formation of the pharmaceutical composition. Preferably, the specific sugar alcohol and process operating conditions are selected so that paracetamol essentially does not melt during formation of the pharmaceutical composition. Suitably, paracetamol may partially dissolve within the molten sugar alcohol; however the majority of the paracetamol is typically dispersed within the molten sugar alcohol. Thus, the sugar alcohol melts and typically enrobes the NSAID salt and paracetamol, and other optional excipients present in the pharmaceutical composition. On cooling the molten sugar alcohol, NSAID salt and paracetamol mixture, an amorphous (i.e. glassy non-crystalline structure) solid phase is formed which may be milled directly into a granule that is suitable for compressing into a pharmaceutical dosage form with minimal tabletting excipients. In other words, the sugar alcohol at least in part loses its crystallinity and acts as a carrier for the NSAID salt and paracetamol. Preferably, when the sugar alcohol is fully melted the sugar alcohol on cooling forms a single continuous phase, namely a single continuous amorphous solid phase i.e. all of the sugar alcohol is essentially amorphous and it is not interrupted by the sugar alcohol having a defined crystalline structure.

Unexpectedly, if the sugar alcohol is fully melted during the preparation of the pharmaceutical composition, then the solidified melt granules typically exhibit improved flow characteristics and are typically easier to compress compared with comparable solidified melt granules formed by partially melting the sugar alcohol. Conveniently, solidified melt granules formed by fully melting the sugar alcohol are typically easier to process, for example they tend not to stick to the punches of a tabletting machine, compared with solidified melt granules formed by partially melting the sugar alcohol. Suitably, the throughput and efficiency of subsequent processing steps (i.e. tabletting process) may be increased significantly by using solidified melt granules where the sugar alcohol is fully melted.

Moreover, solid dosage forms, in particular tablets, formed from solidified melt granules where the sugar alcohol is fully melted are typically more robust and harder than corresponding solid dosage forms formed from solidified melt granules where the sugar alcohol is partially melted. Conveniently, solid dosage forms, in particular tablets, formed from solidified melt granules where the sugar alcohol is fully melted are typically more suited to withstand the further rigours of the manufacturing process (i.e. film coating or sugar coating) compared with solid dosage forms formed by partially melting the sugar alcohol.

Suitably, if the sugar alcohol is fully melted during the preparation of the pharmaceutical composition, then the pharmaceutical composition may exhibit an improved dissolution profile in comparison to a comparable pharmaceutical composition formed by partial melting of the sugar alcohol. In this respect, pharmaceutical compositions which include the sugar alcohol as a single continuous amorphous phase may release a higher concentration of the NSAID salt and/or paracetamol in an aqueous medium over a relatively short period of time compared with a comparable pharmaceutical composition which includes the sugar alcohol in a fully crystalline form or partially crystalline form.

The NSAID salt and the paracetamol may be combined with the molten sugar alcohol, either prior to melting the sugar alcohol or after the melting process, thereby forming a melt mixture comprising the molten sugar alcohol having the NSAID salt and paracetamol contained therein. Preferably, both the NSAID salt and paracetamol are combined with the sugar alcohol prior to melting the sugar alcohol (i.e. a dry mix of the sugar alcohol, NSAID salt and paracetamol is formed and then the sugar alcohol is melted). The NSAID salt is typically insoluble in the sugar alcohol melt and a dispersion of the NSAID salt within the liquid melt is typically produced. Typically, as described hereinafter, the NSAID salt has a melting point far higher than the melting point of the sugar alcohol and the NSAID salt does not melt during formation of the pharmaceutical composition of the present invention. Preferably, by the appropriate choice of sugar alcohol and process conditions, the paracetamol does not melt during formation of the pharmaceutical composition. Conveniently, this permits the NSAID salt and paracetamol to be processed at relatively low temperatures thereby substantially minimising and/or preventing degradation of the NSAID salt and paracetamol.

Preferably, the melt mixture of NSAID salt, paracetamol and molten sugar alcohol is mixed so that the NSAID salt, and any other optional tabletting excipients present in the pharmaceutical composition, is typically uniformly dispersed within the molten sugar alcohol. A uniform mixture is thus produced.

The mixture is allowed to cool by methods hereinafter discussed until a solid is produced. As the mixture cools, it becomes more viscous. The solidified mixture is then formed into melt granules. Thus, as used herein, the term "solidified melt granules" means granules formed by combining the molten sugar alcohol with the NSAID salt and paracetamol, optionally with other tabletting excipients, cooling to a temperature below the melting point of the sugar alcohol and forming the solid mass into granules. The pharmaceutical composition comprises a plurality of such granules.

Thus, the solidified melt granules may be obtainable by fully or partially melting the sugar alcohol. According to a preferred aspect of the present invention the solidified melt granules are obtainable by fully melting the sugar alcohol.

The melt is allowed to solidify in any manner found convenient. This includes both rapid cooling and slow cooling. Preferably, the melt is cooled rapidly (i.e. quenched) as described herein. Typically, this allows the molten sugar alcohol to form a single continuous amorphous phase. For example, the melt may be allowed to cool in a cooled vessel. The melt may be poured onto cooling trays which may be static or continuously moving. Static trays may be placed in cooling cabinets. Moving trays or belts may have additional cooling means, such as cooled water. The cooled melt forms a solid and may be scraped off the belt or collected as it falls off one end of a continuously moving belt.

The solidified melt of the sugar alcohol incorporating the NSAID salt and paracetamol may be formed into granules by a plurality of methods. For example, it may be pulverised into granules. It may be milled and/or sieved. It may also be passed through a spray device such as a spray tower or spray granulator in which the molten material is sprayed from an orifice into a stream of cooled air, allowed to congeal/solidify and then collected. If the melt is extruded, the extrudate maybe cooled and then broken into conveniently sized pieces, followed by milling and or sieving. Alternatively, the extrudate may be extruded through holes and chopped into suitably sized granules for tabletting.

In the preparation of the granular composition, the sugar alcohol is melted. Under pressurised conditions, the sugar alcohol may be melted at a temperature below its normal melting point. Melting may be carried out according to known methods, including for example, heating in a vessel to a temperature above the melting point of the sugar alcohol or by extrusion in a heated extruder. The maximum temperature is determined by the stability of the molten sugar alcohol and ingredients combined therewith. Generally, the higher the temperature, the more quickly the sugar alcohol will melt although this must be balanced by the energy input required to heat the sugar alcohol. For highest efficiency, it is generally envisaged that the sugar alcohol will be heated to not more than 30 °C, preferably 10-30 °C, above its melting point to keep energy costs to a minimum. Although typical operating temperatures are dependent on, amongst other things, the particular sugar alcohol employed as defined herein, a preferred heating range is 80 to 180 °C, more preferably 90 to 170 °C, further preferably 100 to 160 °C, most preferably 110 to 150 °C. If the sugar alcohol is extruded, generally the extruder is heated to a given temperature. In addition, the work on the sugar alcohol by the screw configuration in the extruder will also contribute to melting the sugar alcohol thereby reducing its external applied temperature requirement. Accordingly, the extruder barrel may be heated to a temperature less than the melting point of the sugar alcohol. For example, the normal melting point of xylitol is 95 to 97 °C, however under conditions of force/pressure (such as may be encountered in an extruder or similar processing device), the external applied heat necessary to melt the sugar alcohol may be reduced significantly through the mechanical heat generated by the intense mixed action within the extruder. It is generally envisaged that the extruder will be heated to a temperature not less than 25 °C below the melting point of the sugar alcohol, preferably in the range from 20 °C below the melting point of the sugar alcohol to 30 °C above the melting point of the sugar alcohol, more preferably to a temperature in the range of 20 °C on each side of the melting point of the sugar alcohol. Some extruders allow different zones to be heated to different temperatures in the extruder. These temperatures can be chosen as desired to ensure that the sugar alcohol is fully melted.

Suitably, the sugar alcohol is in the form of a solid at room temperature (i.e. 20 to 25°C) and standard atmospheric pressure. By the term "sugar alcohol" we mean the resultant alcohol formed by reduction of the corresponding mono-and/or poly-saccharides. Typical saccharide materials include sugars such as dextrose and maltose, for example D-sorbitol which may be formed by the reduction of glucose. Such sugar alcohols are typically referred to as "alditols" as they may be formed by reduction of the aldehyde and keto group of the corresponding aldose and ketose sugars respectively.

Preferably, the sugar alcohol is derivable from reduction of a monosaccharide or a disaccharide. More preferably, the sugar alcohol is derivable from reduction of a monosaccharide.

Preferred sugar alcohols derivable by reduction of disaccharides include maltitol (mpt 149 to 152 °C), isomalt (mpt 145 to 150 °C) and lactitol (mpt 95 to 98 °C), of which, maltitol and lactitol are preferred.

Preferred sugar alcohols derivable by reduction of monosaccharides include D-sorbitol (mpt 98 to 100 °C), xylitol (mpt 95 to 97 °C), adonitol (mpt 102 to 104 °C), arabitol (mpt 101 to 104 °C), mannitol (mpt 167 to 170 °C), dulcitol (mpt 188 to 191 °C) and meso-erythritol (mpt 120 to 123 °C). More preferred sugar alcohols derivable by reduction of monosaccharides include D-sorbitol, xylitol, adonitol, arabitol and meso-erythritol. Most preferred sugar alcohols derivable by reduction of monosaccharides include D-sorbitol and xylitol, especially xylitol.

Preferred operating temperatures for melting the above sugar alcohols, preferably by a melt-extrusion process as defined herein, are about 10 °C to 30 °C above the melting point of the particular sugar alcohol so that the sugar alcohol is fully molten. Thus, preferred operating temperatures are as follows: sorbitol between about 108 °C and about 132 °C, xylitol between about 102 °C and about 127 °C, adonitol between about 112 °C and about 134 °C, arabitol between about 111 °C and about 134 °C, mannitol between about 177 °C and about 200 °C, meso-erythritol between about 130 °C and about 153 °C, factitol between about 105 °C and about 128 °C, maltitol between about 159 °C and about 182 °C, and isomalt between about 155 °C and about 180 °C.

Although a mixture of sugar alcohols as defined herein may be used to form the pharmaceutical composition, preferably only a single sugar alcohol is used. Thus, in a preferred embodiment of the pharmaceutical composition the sugar alcohol preferably consists essentially of D-sorbitol or xylitol, especially essentially only xylitol.

Preferably, the sugar alcohol has a melting point of less than or equal to 180 °C, more preferably less than or equal to 170 °C, even more preferably less than or equal to 150 °C, most preferably less than or equal to 120 °C.

Preferably the sugar alcohol has a melting point of greater than or equal to 50 °C, more preferably greater than or equal to 70 °C, most preferably greater than or equal to 90 °C.

The sugar alcohol typically has a melting point which is less than the melting point of the NSAID salt. Preferably, the melting point of the sugar alcohol is at least 40 °C, more preferably at least 60 °C, even more preferably at least 80 °C, most preferably about 100 °C less than the melting point of the NSAID salt.

The sugar alcohol typically has a melting point which is less than the melting point of paracetamol. Suitably the paracetamol does not melt during formation of the pharmaceutical composition. Preferably, the melting point of the sugar alcohol is at least 40 °C, more preferably at least 50 °C, even more preferably at least 60 °C, most preferably at least about 70 °C less then the melting point of paracetamol.

Conveniently, the melt granules may be formed at temperatures substantially less than the melting point of the NSAID salt and paracetamol. In other words, the melt mixture comprising the molten sugar alcohol, the NSAID salt and paracetamol is formed by the application of heat at a temperature which is sufficient to melt the sugar alcohol but not melt the NSAID salt and the paracetamol. Advantageously, such operating conditions typically minimise or prevent degradation of the NSAID salt and paracetamol.

Preferably, the sugar alcohol is present in an amount of less than or equal to 25% by wt, more preferably less than or equal to 20% by wt, more preferably less than or equal to 15% by wt, even more preferably less than or equal to 10% by wt of the granular component of the pharmaceutical composition. Preferably, the sugar alcohol is present in an amount of greater than or equal to 1% by wt, more preferably greater than or equal to 4% by wt, most preferably greater than or equal to about 5% by wt of the granular component of the pharmaceutical composition.

Preferably, the sugar alcohol is present in an amount of less than or equal to 25% by wt, more preferably less than or equal to 20% by wt, even more preferably less than or equal to 15% by wt, most preferably less than or equal to 10% by wt based on the total weight of the pharmaceutical composition. Preferably, the sugar alcohol is present in an amount of greater than or equal to 1% by wt, more preferably greater than or equal to 4% by wt, most preferably greater than or equal to about 5% by wt based on the total weight of the pharmaceutical composition.

The invention allows the formation of a granular composition comprising a variety of NSAID salts, in particular NSAIDS which preferentially inhibit Cox-1.

Suitable types of NSAIDS which preferentially inhibit Cox-1 may be selected from the following categories:
(1) the propionic acid derivatives;
(2) the acetic acid derivatives;
(3) the fenamic acid derivatives;
(4) the biphenylcarboxylic acid derivatives;
(5) the oxicams.

Suitable propionic acid derivatives for use herein include, but are not limited to, ibuprofen, naproxen, benoxaprofen, flurbiprofen, fenoprofen, fenbufen, ketoprofen, indoprofen, pirprofen, carprofen, oxaprozin, prapoprofen, miroprofen, tioxaprofen, suprofen, alminoprofen, tiaprofenic acid, fluprofen, and bucloxic acid. Preferred members of the propionic acid group include ibuprofen, naproxen, flurbiprofen, fenoprofen, ketoprofen and fenbufen, especially ibuprofen.

Suitably acetic acid derivatives for use herein include, but are not limited to, indomethacin, sulindac, tolmetin, zomepirac, diclofenac, fenchlofenac, alchlofenac, ibufenac, isoxepac, furofenac, tiopinac, zidometacin, acemetacin, fentiazac, clidanac and oxipinac. Preferred members of the acetic acid group include tolmetin sodium, zomepinac sodium, sulindac and indomethacin.

The fenamic acid derivatives for use herein include, but are not limited to, mefenamic acid, meclofenamic acid, flufenamic acid, niflumic acid and tolfenamic acid. Preferred members of the fenamic acid group include mefenamic acid and meclofenamic acid.

The biphenylcarboxylic acid derivatives for use herein include, but are not limited to, diflunisal and flufenisal.

The oxicams for use herein include, but are not limited to, piroxicam, sudoxican, isoxicam. A preferred member of this group is piroxicam.

Suitably, the NSAIDS for use in the present invention typically exhibit isomerism. Suitably, all stereoisomers, diastereoisomers, enantiomers and mixtures therefore, including racemic mixtures, of the NSAIDS are embraced by the scope of the present invention.

A highly favoured class of NSAID salts are salts of the propionic acids derivatives.

Preferred salts of propionic acid derivatives, especially 2-aryl propionic acid salts, include salts, of naproxen, flurbiprofen, ibuprofen and ketoprofen, particularly racemic mixtures and S(+)- enantiomers thereof. More preferred 2-aryl propionic acid salts include salts of flurbiprofen and ibuprofen, particularly racemic mixtures and S(+)- enantiomers thereof. Even more preferred 2-aryl propionic acid salts include salts of racemic flurbiprofen and salts of racemic ibuprofen, especially salts of racemic ibuprofen.

The NSAID used in the present invention is in the form of a salt. Representative examples of salts include: alkali metal salts, for example the sodium or potassium salts; alkaline earth metal salts, for example the magnesium or calcium salts; metal salts, for example aluminium salts; amino acid salts, for example the lysine or arginine salts; or, amine salts, for example meglumine salt.

Preferred salts include the alkali metal salts, the alkaline earth metal salts, amine salts and the amino acid salts. More preferred salts include the alkali metal salts, amine salts and the amino acid salts. Most preferred salts include the alkali metal salts, particularly the sodium or potassium salts, especially the sodium salt.

Suitably, a highly preferred NSAID salt for use in the present invention is the sodium salt of racemic ibuprofen or the sodium salt of S(+)-ibuprofen. Most preferably, the NSAID salt comprises the sodium salt of racemic ibuprofen.

Suitably, the granular composition may comprise one or more different NSAID salts as defined herein. Preferably, however, the granular composition comprises a single NSAID salt. Most preferably, the granular composition comprises a single NSAID salt in a single enantiomeric form or as a racemic mixture i.e. S(+)-ibuprofen only or racemic ibuprofen only. Moreover, as described hereinafter, the pharmaceutical composition may include one or more further pharmaceutically active agents in addition to the NSAID salt and paracetamol. However, a highly preferred pharmaceutical composition of the present invention includes NSAID salts and paracetamol as the only pharmaceutically active agents, most preferably a single NSAID salt as defined herein and paracetamol.

The NSAID salt may be in an anhydrous or hydrated form. Preferably, the NSAID salt is in a hydrated form. In this respect, the dihydrate of the sodium salt of racemic ibuprofen is a particularly preferred NSAID salt.

The NSAID salts typically having a melting point of between about 150 °C and about 270 °C, preferably between about 170 °C and about 260 °C. In this respect, sodium ibuprofen dihydrate has a melting point of about 200 °C, naproxen sodium has a melting point of about 250 to 251 °C, and ibuprofen lysinate has a melting point of about 177 to 180 °C.

The proportion of NSAID salt in the pharmaceutical composition will depend on the dose desired for therapeutic effect. Low dose drugs, such as salts of flurbiprofen and ketoprofen may form as little as 10% by weight of the granular component of the pharmaceutical composition in order to provide that a pharmaceutical dosage form (i.e. tablet) produced from the composition is not too small. However, a preferred feature of the invention is that high dose NSAID salts, such as salts of ibuprofen, can be formulated into smaller dosage forms. Accordingly, the NSAID salt typically forms greater than or equal to 20% by wt, preferably greater than or equal to 25% by wt, more preferably greater than or equal to 30% by wt of the granular component of the pharmaceutical composition. Suitably, the NSAID salt typically forms less than or equal to 60% by wt, more preferably less than or equal to 50% by wt, most preferably less than or equal to 45% by wt of the granular component of the pharmaceutical composition.

Preferably, the NSAID salt is present in an amount of greater than or equal to 20% by wt, more preferably greater than or equal to 25% by wt, even more preferably greater than or equal to 30% by wt based on the total weight of the pharmaceutical composition of the present invention.

Preferably, the NSAID salt is present in an amount of less than or equal to 55% by wt, more preferably less than or equal to 50% by wt, most preferably less than or equal to 45% by wt based on the total weight of the pharmaceutical composition of the present invention.

Preferably, the percent by weight ratio of NSAID salt to sugar alcohol in the granular component of the pharmaceutical composition is 20:1 to 2:1, more preferably 10:1 to 2:1, most preferably 9:1 to 3:1.

Preferably, the percent by weight ratio of NSAID salt to sugar alcohol in the pharmaceutical composition is 20:1 to 2:1, more preferably 10:1 to 2:1, most preferably 9:1 to 3:1.

Unit dosages for effective therapy are known to those skilled in the art for each NSAID. For example, they may comprise the NSAID to an extent of 5 mg, 10 mg, 12.5 mg, 25 mg, 50 mg, 100 mg, 150 mg, 200 mg, 250 mg, 300 mg, 350 mg, 400 mg, 500 mg, 600 mg and 800 mg. Where derivatives are employed, normally the precise unit dosages are chosen to give the equivalent NSAID doses given above. For the treatments described herein the maximum daily dose of ibuprofen is generally 3200 mg. A single unit daily dose may be 100 mg. Preferred unit doses are in the range 100-400 mg, more preferably 100-300 mg and especially 200 mg ibuprofen. The maximum daily dose of flurbiprofen is generally 300 mg. A single unit dose may be 12.5 mg. Preferred unit doses are in the range 12.5-150 mg, more preferably 25-100 mg and especially 50 mg flurbiprofen. The maximum daily dose of naproxen is generally 1500 mg. A single unit daily dose may be 125 mg. Preferred unit doses are in the range 220-750 mg, more preferably 220-500 mg and especially 220-250 mg naproxen. The maximum daily dose of ketoprofen is generally 200 mg. A single unit daily dose may be 25 mg. Preferred unit doses are in the range 25-100 mg, more preferably 25-75 mg and especially 50 mg ketoprofen.

Preferably, the paracetamol forms greater than or equal to 30% by wt, more preferably greater than or equal to 35% by wt, even more preferably greater than or equal to 40% by wt, most preferably greater than or equal to 45% by wt of the granular component of the pharmaceutical composition.

Preferably, the paracetamol forms less than or equal to 70% by wt, more preferably less than or equal to 65% by wt, most preferably less than or equal to 60% by wt of the granular component of the pharmaceutical composition.

Preferably, the paracetamol forms greater than or equal to 30% by wt, more preferably greater than or equal to 35% by wt, even more preferably greater than or equal to 40% by wt, most preferably greater than or equal to 45% by wt based on the total weight of the pharmaceutical composition.

Preferably, the paracetamol forms less than or equal to 70% by wt, more preferably less than or equal to 65% by wt, most preferably less than or equal to 60% by wt based on the total weight of the pharmaceutical composition.

Unit dosages for effective therapy are known to those skilled in the art for paracetamol. For example, they may comprise paracetamol to an extent of 100 mg, 150 mg, 200 mg, 250 mg, 300 mg, 350 mg, 400 mg, 450 mg, 500 mg, 550 mg, 600 mg, 650 mg, 700 mg and 800 mg. Preferred unit doses are in the range of 100 to 650 mg, more preferably 200 to 550 mg, most preferably 250 to 500 mg.

Preferably, the percent by weight ratio of NSAID salt to paracetamol in the granular component of the pharmaceutical composition is 1:5 to 3:1, more preferably 1:3 to 2:1, most preferably 1:2 to 1:1.

Preferably, the percent by weight ratio of NSAID salt to paracetamol in the pharmaceutical composition is 1:5 to 3:1, more preferably 1:3 to 2:1, most preferably 1:2 to 1:1.

Preferably, the pharmaceutical composition further includes one or more disintegrating agents. The disintegrating agent may be present in the granular component and/or present as an extra-granular component. Preferably, the disintegrating agent is present within the granular component, even more preferably the disintegrating agent is only present within the granular component. If a disintegrating agent is incorporated into the molten sugar alcohol having the NSAID salt contained therein and intimately combined therewith, the mixture cooled and milled to produce a granule, a pharmaceutical composition capable of tabletting with minimum tabletting excipients and having advantageous tabletting, disintegration and dissolution properties is provided. The disintegrating agent has the effect of causing a solid dosage form, such as a tablet, formed from the pharmaceutical formulation to disintegrate under the conditions found in the gastro-intestinal tract. Examples of disintegrating agents include one or more of wheat starch, maize, starch, potato starch, sodium starch glycolate, low-substituted hydroxypropyl cellulose, alginic acid, cross-linked polyvinylpyrrolidone, magnesium aluminium silicate and croscarmellose sodium. Preferred disintegrating agents are those which swell on the action of water thus causing the ingredients in the pharmaceutical composition to be pushed apart and out into the aqueous disintegration medium. Preferred disintegrating agents comprise one or more of croscarmellose sodium and sodium starch glycolate, especially croscarmellose sodium.

Preferably, the disintegrating agent is present in an amount of less than or equal to 20% by wt, more preferably less than or equal to 15% by wt, even more preferably less than or equal to 10% by wt of the granular component of the pharmaceutical composition. Preferably the disintegrating agent is present in an amount of greater than or equal to 1% by wt, more preferably greater than or equal to 2% by wt, most preferably greater than or equal to 3% by wt of the granular component of the pharmaceutical composition.

Preferably, the disintegrating agent is present in an amount of less than or equal to 20% by wt, more preferably less than or equal to 15% by wt, most preferably less than or equal to 10% by wt based on the total weight of the pharmaceutical composition. Preferably, the disintegrating agent is present in amount of greater than or equal to 1% by wt, more preferably greater than or equal to 2% by wt, most preferably greater than or equal to 3% by wt based on the total weight of the pharmaceutical composition.

Preferably, the percent by weight ratio of NSAID salt to disintegrating agent in the granular component of the pharmaceutical composition is 20:1 to 2:1, more preferably 15:1 to 5:1, most preferably 12:1 to 7:1.

Preferably, the percent by weight ratio of paracetamol to disintegrating agent in the granular component of the pharmaceutical composition is 20:1 to 2:1, more preferably 15:1 to 5:1, most preferably 12:1 to 7:1.

Suitably, when the granular component includes a disintegrating agent, the percent by weight ratio of the sugar alcohol to the disintegrating agent in the granular component is preferably 5:1 to 1:5, most preferably approximately 3:1 to 1:3, most preferably 2:1 to 1:2.

According to a preferred aspect, the present invention provides a pharmaceutical composition comprising a granular component comprising a plurality of solidified melt granules of a sugar alcohol and incorporating an NSAID salt, paracetamol and disintegrant contained therein. Preferably, the pharmaceutical composition comprises a granular component comprising a plurality of melt granules of a molten sugar alcohol incorporating a NSAID salt, paracetamol and a disintegrating agent uniformly dispersed therein. Preferably, croscarmellose sodium or sodium starch glycolate (although any known disintegrant may be used), may be the sole excipient incorporated in the granular component. Alternatively, the granular component may contain additional excipients such as a diluent and optionally a surfactant. Accordingly, the granular component may consist essentially of (i.e. greater than 98% by weight of the granular component) a sugar alcohol, NSAID salt, paracetamol and a disintegrating agent or it may consist essentially of a sugar alcohol, a NSAID salt, paracetamol, a disintegrating agent, a diluent and optionally a surfactant. Thus, the diluent and optional surfactant may be combined with the disintegrant, NSAID salt, paracetamol and molten sugar alcohol.

Preferably, the granules comprise a single continuous amorphous phase of the sugar alcohol. More preferably, the pharmaceutical composition is in the form of a solid dosage form for oral administration. Even more preferably, the pharmaceutical composition is in the form of a tablet, most preferably a compressed tablet, especially a compressed tablet composition which is adapted to release the medicament in the stomach or gastro-intestinal tract.

Although not necessary for the carrying out the present invention, if desired the pharmaceutical composition (i.e. the compressed tablet) may comprise additional excipients.

For example, the pharmaceutical composition may comprise a proportion of a water-soluble or water-insoluble compressible diluent. Suitable water-soluble diluent materials include sugars (such as sucrose, fructose, lactose, dextrose), cyclodextrin, maltodextrin and salts of organic acids (e.g. sodium citrate and potassium citrate). The sugar alcohol present in the solidified melt granules may function, amongst other things, as a water soluble diluent. Lactose, sodium citrate and potassium citrate are particularly preferred water-soluble diluents. Suitable water-insoluble diluent materials include cellulose derivatives (such as microcrystalline cellulose) starch and derivatives thereof (such as pre-gelatinised starch), dicalcium phosphate, tricalcium phosphate, calcium sulphate, calcium carbonate. Microcrystalline cellulose and dicalcium phosphate are preferred water insoluble diluents. In a pharmaceutical composition adapted to disperse in water prior to administration, the level of diluent may be quite high, for example up to 50% (such as 0-50% w/w, preferably 0-40% w/w) by weight based on the total weight of the composition, in order to achieve the desired dispersing properties. Preferably, when the pharmaceutical composition is in the form of a solid dosage form for oral administration (i.e. a compressed tablet), the diluent does not form greater than 30% by weight of the pharmaceutical composition (e.g. 0-25% w/w), as it adds to the costs of the composition and to production costs. Thus, to minimise costs it may be preferred that the diluent is added to the pharmaceutical composition in an amount of 0-20% by weight of the pharmaceutical composition, more preferably 0-10% w/w. If present, it may be preferably used to an extent of 0.1-25% by weight based on the total weight of the pharmaceutical composition, more preferably 0.1-20% by weight, further preferably 1-15% w/w and most preferably 4-15% by weight of the pharmaceutical composition. The diluent may form part of the granular component and/or it may be present as an extra-granular component. Preferably, the diluent, especially microcrystalline cellulose and dicalcium phosphate, are present within an extra-granular component.

The pharmaceutical composition, particularly the granular component, may also include a surfactant, in an amount appropriate to the properties of the surfactant, preferably 0.05-10% by weight based on the total weight of the pharmaceutical composition. Preferred surfactants are sodium lauryl sulphate and poloxamer. They may be used to an extent of 0.05-8% by weight (preferably 0.1-5% by weight, more preferably 0.2-2% by weight) based on the total weight of the pharmaceutical composition.

The melt granules in the granular composition preferably have a mean particle size in the range 10-2000µm, more preferably 50-1000µm and most preferably 100-400µm. Valuable results are achieved when the bulk density of the melt granules is in the range 0.1-1gml⁻¹, more preferably 0.3-0.6gml⁻¹. Further preferred properties are obtained when the tapped density is in the range 0.3-0.7gml⁻¹ (more preferably 0.4-0.6 gml⁻¹).

In a pharmaceutical composition according to the present invention, it is preferred that the granular component comprising the plurality of melt granules is combined with an extra-granular component. Preferably the pharmaceutical composition comprises a granular component in an amount of 60-99.95%, more preferably 70-99.9% by weight, especially 75-99.9% by weight, particularly 80-99.9% by weight of the pharmaceutical composition and 0.05-40% extra-granular component, preferably 0.1-30%, especially 0.1-25%, particularly 0.1-20% by weight of the pharmaceutical composition.

The extra-granular component comprises the ingredients incorporated in the pharmaceutical composition which are not contained in the solidified melt granules. They may be mixed with the melt granules simultaneously or at sequential stages in the process to prepare unit dosages. A particular advantage of the present invention is preferably that all the ingredients of the extra-granular component are combined with the granular component at the same time and also there does not have to be significant processing of the ingredients in the extra-granular component prior to combining with the granular component. The pharmaceutical composition typically comprises a uniform mixture of granular component and extra-granular component, and it may be compressed into tablets so the extra-granular component is suitably distributed evenly throughout the tablet.

A preferred pharmaceutical composition of the present invention comprises:
a) 60-99.5% granular component by weight of the composition, said granular component incorporating 0.005-1 parts by weight disintegrant per part by weight of non-steroidal anti-inflammatory drug; and
b) 0.05-40% extra-granular component by weight of the composition.

Preferably, the pharmaceutical composition includes a wicking agent. As used in this specification, the term "wicking agent" refers to any excipient that forms capillary pathways within a compact, such as a tablet, such that when the compact is placed in an aqueous environment liquid is drawn through the pathways by capillary action, disintegration of the compact occurs as interparticulate bonds are ruptured by the ingress of liquid. The wicking agent is insoluble in water. The wicking agent may be present in the granular component and/or the extra-granular component. Preferably, the wicking agent is present in the extra-granular component. Most preferably, the wicking agent is present only in the extra-granular component.

By "insoluble in water", we mean that more than 10,000 ml of water is required to produce a solution of 1 gram of solid at a temperature in the range of 15-25%.

In one preferred aspect of the present invention there is provided a pharmaceutical composition comprising a granular component comprising a plurality of solidified melt granules of a sugar alcohol incorporating a NSAID salt and paracetamol uniformly contained therein combined with an extra-granular component comprising a wicking agent as defined herein. Preferably, the granular component further comprises a disintegrant.

Suitably, the wicking agent is present in an amount of 0.1-15% by weight (preferably 0.1-8% by weight, preferably 0.1-5% by weight, more preferably 0.2-3% by weight) based on the total weight of the pharmaceutical composition. Said insoluble wicking agent is selected from inorganic materials, starch materials, cellulose materials such as hydroxyethylcellulose (HEC), hydroxypropylcellulose (HPC), hydroxypropylmethyl cellulose (HPMC), and mixtures thereof. Preferably the inorganic material comprises silicon dioxide, PTFE powder, alkali metal silicates, alkaline earth metal silicates, alkali metal carbonates and bicarbonates and alkaline earth metal carbonates. Examples include sodium carbonate, sodium bicarbonate, potassium carbonate, magnesium carbonate, calcium carbonate, PTFE powder, sodium silicate, potassium silicate, magnesium silicate and calcium silicate. Preferably the starch material comprises starches such as potato starch, maize starch, rice starch, tapioca starch and starch derivatives including modified starches such as pre-gelatinised starch. More preferably, the wicking agent comprises at least one of silicon dioxide and/or alkaline earth metal carbonates, especially calcium carbonate, talc, maize starch and pre-gelatinised starch. Most preferably, the wicking agent comprises silicon dioxide.

Silicon dioxide is insoluble in water and suitably has a surface area greater than 50 m²g⁻¹, more preferably greater than 100 m²g⁻¹, especially in the range 150-250 m²g⁻¹. Most preferably the silicon dioxide is colloidal silicon dioxide (especially having a mean particle size less than 50nm such as 5-40nm), most preferably anhydrous colloidal silicon dioxide. The tapped density of the silicon dioxide is preferably in the range 0.01-0.2gcm⁻².

The wicking agent, for example silicon dioxide, is preferably incorporated in the composition to an extent of 0.05-5.0% by weight (preferably 0.1-3% by weight, more preferably 0.2-1% by weight) based on the total weight of the pharmaceutical composition.

The silicon dioxide may be incorporated in the melt granules. If silicon dioxide is incorporated in the melt granules, it is typically used to an extent of 0.1-1%, more preferably 0.2-0.8% by weight based on the total weight of pharmaceutical composition.

Preferably, the silicon dioxide is present in the extra-granular component. Further preferably, the silicon dioxide is present in the extra-granular component to an extent of 0.1-3%, more preferably 0.2-2% by weight based on the total weight of the pharmaceutical composition.

A preferred pharmaceutical composition comprises a granular component as defined herein and an extra-granular component including silicon dioxide.

It is surprising that the small amounts of a wicking agent such as silicon dioxide has the effect of causing the composition to disperse so quickly in aqueous conditions, especially in acidic conditions (such as are found in the stomach) leading to a high percentage of the NSAID being dissolved or dispersed within a relatively short period.

The present invention preferably provides the use of a water-insoluble wicking agent as defined herein, particularly silicon dioxide, in an extra-granular component combined with a granular component as defined herein in a compressed composition, said granular component comprising a plurality of solidified melt granules of a sugar alcohol having an NSAID salt, paracetamol and optionally one or more excipients contained therein, wherein the wicking agent enhances the dispersion of the compressed composition in aqueous conditions. The granules preferably incorporate a disintegrant and optionally a diluent uniformly dispersed therethrough. The composition preferably comprises 0.05 to 10%, preferably 0.1 to 5% of the wicking agent, for example 0.1 to 5% of silicon dioxide by weight of the formulation.

Optionally a lubricant may be incorporated in the pharmaceutical composition. The lubricant may be incorporated in the granular component and/or in the extra-granular component. Preferably, the lubricant is incorporated in the extra-granular component for mixing with the granular component. Conventional lubricants for ibuprofen tablets may be used for example stearic acid, sodium lauryl sulphate, polyethylene glycol, hydrogenated vegetable oil, sodium stearyl fumarate, magnesium stearate or calcium stearate. These may be present in an amount from 0.05 to 5% by weight, preferably 0.1 to 3.0% by weight based on the total weight of the pharmaceutical composition.

An advantageous pharmaceutical composition according to the present invention comprises a granular component as defined herein and an extra-granular component comprising silicon dioxide and a lubricant and optionally a diluent. The extra-granular component may form an intimate admixture with said granular component prior to compression into a tablet.

Other conventional tabletting excipients known to the person skilled in the art may be incorporated in the pharmaceutical composition according to the present invention as desired, although it will be appreciated that a prime advantage of the present invention is that the number of excipients necessary to achieve a quickly disintegrating dosage form, such as a compressed tablet, with good dissolution characteristics is minimal.

The present invention also provides a formulation comprising a NSAID drug, paracetamol and at least one further pharmacologically active ingredient and/or enhancing agent. The further pharmaceutically active ingredient may be present in the granular component or in the extra-granular component. Formulations comprising an additional pharmaceutically active ingredient and/or enhancing agent can be provided in any form suitable for patient consumption but are preferably provided in the form of a tablet.

Thus, for example, the dosage form may include any other ingredient commonly used in a composition useful to treat pain, inflammation and/or fever, for example caffeine or another xanthine derivative, another analgesic, for example codeine, a skeletal muscle relaxant: an antihistamine (e.g. acrivastine, astemizole, azatadine, azelastine, bromodiphenhydramine, brompheniramine, carbinoxamine, cetirizine, chlorpheniramine, cyproheptadine, dexbromopheniramine, dexchloropheniramine, diphenhydramine, ebastine, ketotifen, lodoxamide, loratidine, levocabastine, mequitazine, oxatomide, phenindamine, phenyltoloxamine, pyrilamine, setastine, tazifylline, temelastine, terfenidine, tripelennamine or triprolidine (preferably non-sedating antihistamines are employed)); a decongestant (e.g. pseudoephedrine, phenylpropanolamine and phenylephrine); a cough suppressant (e.g. caramiphen, codeine or dextromethorpan); an expectorant (e.g. guaifenesin, potassium citrate, potassium guaiacolsuphonate, potassium sulphate and terpin hydrate); an anti-ulcer histamine antagonist (e.g. misoprostol); and/or an anti-nausea drug (e.g. domperidone).

Such extra active ingredients and/or enhancing agents may be incorporated in the melt granules or in the extra-granular component which is combined with the melt granule prior to formulation into a compressed tablet. Reference may be made to MIMS and the Physicians Desk Reference for guidelines as to a suitable dosage. It is generally expected that such other active ingredients will form 0.1-50% w/w of the formulation, for example 5-25% w/w.

The ratio of NSAID salt to the further pharmacologically active ingredient or ingredients will depend on the proportion of the NSAID salt in the dosage form. Thus, depending on the dosage of the drug, it can be expected to fall in the range 20:1 to 1:100, conveniently 5:1 to 1:40. For relatively high dose drugs such as ibuprofen, the ratio of NSAID salt to further pharmacologically active ingredient or ingredients may preferably be in the range 1:5 to 1:25, more preferably 1:6 to 1:20. For relatively low dose drugs such as flurbiprofen, the ratio of NSAID salt to further pharmacologically active ingredient may suitably be 10:1 to 1:10, preferably 1:4 to 4:1 parts by weight.

If the further pharmacologically active ingredient or ingredients is present in the granular component of the formulation it is typically combined with the sugar alcohol, NSAID salt and paracetamol in the solid state. Typically, the mixture is then heated to melt at least the sugar alcohol. A liquid is formed. The further pharmacologically active ingredient or ingredients may be soluble or insoluble in the molten sugar alcohol, accordingly, a solution or a dispersion of the further pharmacologically active ingredient or ingredients within the liquid sugar alcohol melt is produced on melt-extruding the combination. Generally, the further pharmacologically active ingredient or ingredients have a higher melting point than the sugar alcohol in which they are incorporated. If they have a lower melting point, the solidified melt will be a combined melt of the sugar alcohol with the low melting point active. If the further pharmacologically active ingredient or ingredients has a melting point higher than that of the sugar alcohol it is intimately mixed with the sugar alcohol in the granular composition and is present as a uniform or homogeneous solid solution or dispersion in the solidified sugar alcohol melt. The liquid melt is cooled until solidified melt is formed. As the mixture cools, it becomes more viscous. The mixture is allowed to cool by methods hereinafter discussed until a solid is produced. The melt granules may be formed before, during or after the sugar alcohol solidifies.

Thus, as used in relation to this aspect of the invention, "solidified melt granules" means granules formed by combining the sugar alcohol, NSAID salt and paracetamol in solid state with a further pharmacologically active ingredient or ingredients, melting the sugar alcohol, cooling and forming the mixture into solidified melt granules. Alternatively, the further pharmacologically active ingredient or ingredients may be mixed with the sugar alcohol once molten.

The further pharmacologically active ingredient or ingredients may be the sole ingredient incorporated within the sugar alcohol melt granules including the NSAID salt and paracetamol or it may be combined with a disintegrant and/or a diluent and optionally a surfactant and other tabletting excipients. Accordingly, in one preferred embodiment, the granules may comprise greater than 80% w/w of the NSAID salt, paracetamol and further pharmacologically active ingredient or ingredients. Preferred melt granules comprise a sugar alcohol, a NSAID salt, paracetamol, further pharmacologically active ingredient or ingredients, a disintegrant and optionally a surfactant and/or a diluent. Further preferred melt granules consist essentially of (98-100% w/w) the combination of a sugar alcohol, a NSAID salt, paracetamol, further pharmacologically active ingredient or ingredients and a disintegrant. Further preferred melt granules consist essentially of a sugar alcohol, a NSAID salt, paracetamol, further pharmacologically active ingredient or ingredients, a disintegrant and a surfactant. A further preferred granular component consists essentially a sugar alcohol, a NSAID salt, paracetamol, further pharmacologically active ingredient or ingredients, a disintegrant, a surfactant and a diluent.

A preferred pharmaceutical composition, especially a compressed tablet composition, comprises an intimate mixture of:
a) a granular component comprising a plurality of solidified melt granules of a sugar alcohol incorporating a NSAID salt and paracetamol uniformly contained therein; and
b) 0.05 to 5.0% by weight of an insoluble wicking agent based on the total weight of the pharmaceutical composition.

A further preferred pharmaceutical composition, especially a compressed tablet composition, comprises an intimate mixture of:
a) a granular component comprising a plurality of solidified melt granules of a sugar alcohol incorporating a NSAID salt, paracetamol and a disintegrant uniformly dispersed therein; and
b) 0.05 to 5% by weight of an insoluble wicking agent based on the total weight of the pharmaceutical composition.

Preferably, as mentioned herein, the insoluble wicking agent is present within an extra-granular component. More preferably, the insoluble wicking agent comprises silicon dioxide.

In a further preferred pharmaceutical composition, for example a compressed tablet, there is provided an intimate mixture of:
a) a granular component comprising a plurality of solidified melt granules of a sugar alcohol incorporating paracetamol, a NSAID salt, preferably an ibuprofen salt, and a disintegrant, preferably croscarmellose sodium, wherein the sugar alcohol is present in an amount of 1 to 25% by weight of the granular component, the NSAID salt is present in an amount of 20 to 60% by weight of the granular component, the disintegrant is present in an amount of 1 to 25% by weight of the granular component, and the paracetamol is present in an amount of 30 to 70% by weight of the granular composition;
b) 0.05 to 5.0% by weight silicon dioxide based on the total weight of the pharmaceutical composition; and optionally
c) a lubricating agent and/or a diluent.

In a further preferred pharmaceutical composition, for example a compressed tablet, there is provided an intimate mixture comprising:
a) 60 to 99% by weight, preferably 70 to 99% by weight, more preferably 85 to 99% by weight based on the total weight of the pharmaceutical composition of a granular component comprising:
   a plurality of solidified melt granules of a sugar alcohol incorporating paracetamol, a NSAID salt, preferably sodium or potassium ibuprofen, a disintegrant and optionally a diluent uniformly dispersed therein, said sugar alcohol being present in an amount of 1 to 25% by weight of the granular component, said NSAID salt being present in an amount of 20 to 60% by weight of the granular component, said disintegrant being present in an amount of 1 to 25% by weight of the granular component, the paracetamol being present in an amount of 30 to 70% by weight of the granular component, and said diluent being present in an amount of 0 to 20% by weight of the granular component;
b) 1 to 40% by weight, preferably 1 to 30% by weight, more preferably 1 to 15% by weight based on the total weight of the pharmaceutical composition of an extra-granular component comprising:
   0.05 to 5% by weight of a wicking agent, especially silicon dioxide, based on the total weight of the pharmaceutical composition.

Preferably, the pharmaceutical composition, especially the extra-granular component, further includes 0.05 to 5% by weight based on the total weight of the pharmaceutical composition of one or more lubricants as defined herein, especially a lubricant selected from stearic acid or a salt thereof i.e. sodium stearate or magnesium stearate.

Alternatively or additionally, the pharmaceutical composition, especially the extra-granular component, further comprises 0.1 to 25% by weight, more preferably 4 to 15% by wt based on the total weight of the pharmaceutical composition of one or more diluents as defined herein, especially a diluent selected from microcrystalline cellulose or dicalcium phosphate.

Preferably, the sugar alcohol is selected from D-sorbitol or xylitol, especially xylitol.

A still further preferred pharmaceutical composition comprises an intimate mixture comprising:
a) 80 to 99% by weight based on the total weight of the pharmaceutical composition of a granular comment comprising:
   a plurality of solidified melt granules of xylitol or D-sorbitol, incorporating paracetamol, an NSAID alkali metal salt, preferably sodium or potassium ibuprofen, and a disintegrant, preferably sodium croscarmellose sodium, uniformly contained therein, wherein:
      (i) the xylitol or D-sorbitol is present in an amount of 4 to 15% by weight based on the total weight of the pharmaceutical composition;
      (ii) the NSAID alkali metal salt is present in an amount of 25 to 45% by weight based on the total weight of the pharmaceutical composition;
      (iii) the paracetamol is present in an amount of 40 to 60% by weight based on the total weight of the pharmaceutical composition; and
      (iv) the disintegrant is present in an amount of 3 to 10% by weight based on the total weight of the pharmaceutical composition; and
b) 1 to 20% by weight of an extra-granular component comprising:
   (v) 0.1 to 3% by weight of an insoluble wicking agent, especially silicon dioxide, based on the total weight of the composition;
   (vi) 0.05 to 5% by weight of a lubricant, especially stearic acid or a salt thereof, based on the total weight of the composition; and
   (vii) 1 to 15% by weight of a diluent, especially microcrystalline cellulose or dicalcium phosphate, based on the total weight of the composition.

Preferably, the sum of components (i) to (vii) being greater than 99% by weight of the composition.

Most preferably, the granular component consists essentially of (i.e. greater than 98% by wt of the granular component) of xylitol or D-sorbitol, sodium ibuprofen, paracetamol and croscarmellose sodium.

NSAID salts and derivatives thereof are primarily anti-inflammatory, analgesic and anti-pyretic agents but have also been proposed for other therapeutic uses, including the treatment of periodontal bone loss, pruritus and Alzheimer's disease. Paracetamol possesses analgesic and anti-pyretic activity. The pharmaceutical composition of the present invention are therefore indicated for use in the treatment of all therapeutic uses for which cyclooxygenase inhibitors are effective, including rheumatoid arthritis, osteoarthritis, ankylosing spondylitis, seronegative arthropathies, periarticular disorders and soft tissue injuries. They may also be used in the treatment of postoperative pain, postpartum pain, dental pain, dysmenorrhoea, headache, migraine, rheumatic pain, muscular pain, backache, neuralgia and/or musculoskeletal pain or the pain or discomfort associated with the following: respiratory infections, colds or influenza, gout or morning stiffness.

Accordingly, in another aspect of the present invention there is provided a pharmaceutical composition according to the present invention for use in the treatment of pain and/or inflammation and/or fever. Furthermore, the invention also provides a method of treating pain and/or inflammation and/or fever comprising the administration of a composition according to the present invention to a mammal in need thereof.

Preferably, the pharmaceutical composition is in the form of a unit dose for oral administration. The unit dose may be swallowed, dispersed in water prior to ingestion or adapted to disintegrate in the mouth. Preferably, the unit dose is adapted to release the NSAID salt and paracetamol in the stomach or the gastro-intestinal tract. Most preferably, the unit dose is swallowed by a patient in need thereof.

Suitable unit doses include compressed tablets, chewable tablets, effervescent formulations, trouches. Most preferably, the unit dose is in the form of a compressed tablet, especially a non-effervescent compressed tablet.

Thus, according to a further preferred aspect, the present invention provides a compressed tablet comprising a pharmaceutical composition as defined herein. Although, the compressed tablet may be swallowed or dispersed in water prior to administration, preferably the compressed tablet is swallowed and adapted to release the NSAID salt and paracetamol in the stomach or gastro-intestinal tract.

The present invention also provides a process for producing the pharmaceutical composition of the present invention comprising the steps of:
a) forming a melt mixture comprising said molten sugar alcohol incorporating said NSAID salt and paracetamol therein, optionally with one or more additional excipients that may be present in the granules; and
b) forming the melt mixture into solidified melt granules.

Preferably, step (a) takes place in a melt extruder. Preferably, in step (b) the melt mixture is cooled to form a solidified melt and the solidified melt is formed into a plurality of melt granules.

Components in addition to the NSAID salt, paracetamol and sugar alcohol that may be present in the solidified melt granules are described above. Such components include but are not limited to disintegrants, additional pharmaceutically active agents, insoluble wicking agents, diluents and lubricants.

In the process of the present invention, the sugar alcohol, paracetamol and NSAID salt and any additional excipient to be included in the melt granules may be mixed in the solid state prior to melting the sugar alcohol. Alternatively, the additional component(s) of the melt granules may be added to the melt mixture comprising said molten sugar alcohol having said NSAID salt and paracetamol contained therein. Processes in which one or more additional components are mixed with the sugar alcohol, paracetamol and NSAID salt prior to melting the sugar alcohol and in which one or more further additional components are added to the melt mixture of the molten sugar alcohol, paracetamol and NSAID salt are also within the scope of the present invention. An especially preferred method involves combining the sugar alcohol, paracetamol and NSAID salt in the solid state, along with any additional excipients to be included in the melt granules, and then melting the sugar alcohol.

The granular composition may be prepared in accordance with the present invention by a simple cost-efficient manufacturing process on a large scale. Formulations prepared from a pharmaceutical composition according to the present invention have been found to be stable on storage and to have advantageous dissolution properties. The formulation may be tabletted without sticking or capping during the tabletting process to provide a dosage form having suitable hardness properties combined with advantageous disintegration properties. Furthermore, the poor taste associated with certain NSAIDS is significantly improved.

The above-mentioned process may be carried out in a number of ways. In one method, the sugar alcohol is heated in a suitable vessel until molten. The NSAID salt and paracetamol may then be added to the molten mass and thoroughly combined therewith to form a uniform mixture. Optional additional excipients may be blended into the melt mixture simultaneously or sequentially. The molten mixture may then be discharged into an appropriate cooling system, for example a cooled belt which may continuously rotate and deliver the cooled melt to a comminuting device such as a scraper bar and/or a mill.

In a further process, the sugar alcohol may be combined with the NSAID salt, paracetamol and any additional excipients which may be present in the granules, e.g. a disintegrant or diluent, and then heated together until said sugar alcohol is fully molten. In yet a further process the sugar alcohol, paracetamol and the NSAID salt are combined and heated together until said sugar alcohol is fully molten and any excipient is uniformly blended with the mixture.

In another method, the sugar alcohol, paracetamol and NSAID salt, and any additional excipients which may be present in the granules, are fed into an extruder type system (preferably having first been combined by blending together). The materials are heated and mixed in the extruder until the sugar alcohol is fully molten and a uniform mixture is produced. The sugar alcohol, paracetamol and NSAID salt and any additional excipients are extruded and the extrudate cooled. Preferably, the sugar alcohol, paracetamol and NSAID salt and any additional excipients are extruded in a twin screw extruder. The hot mass (comprising the sugar alcohol, paracetamol and NSAID salt and any additional component) extruded forms an agglomerated mass which may be collected and, if desired, milled to form granules.

In a further method, after heating or heat-extrusion the sugar alcohol, paracetamol and NSAID salt and any additional excipients may be cooled by feeding to a spray tower dryer in which the molten mass is sprayed into the path of a stream of cold air and the dried solid mass collected.

In the preparation of the granular component, the sugar alcohol is melted. Under pressurised conditions, the sugar alcohol may be melted at a temperature below its normal melting point. Melting may be carried out according to known methods, including for example, heating in a vessel to a temperature above the melting point of the sugar alcohol or by extrusion in a heated extruder. Under conditions of pressure, the sugar alcohol may be melted at a temperature below its normal melting point. The maximum temperature is determined by the stability of the molten sugar alcohol, paracetamol and NSAID salt and further optional ingredients combined therewith. The sugar alcohol may be heated to any convenient temperature. Generally, the higher the temperature, the more quickly the sugar alcohol will melt although this must be balanced by the energy input required to heat the drug. For highest efficiency, it is generally envisaged that the sugar alcohol will be heated to not more than 50°C, preferably not more than 30°C, most preferably 10-30°C, above its melting point to keep energy costs to a minimum.

If the sugar alcohol, paracetamol and NSAID salt are extruded, generally the extruder is heated to a given temperature. In addition, the work on the sugar alcohol by the screw configuration in the extruder will also contribute to melting the sugar alcohol thereby reducing its external applied temperature requirement. Accordingly the extruder barrel may be heated to a temperature less than the melting point of the sugar alcohol. For example, the normal melting point of xylitol is 95-97°C, however under conditions of force/pressure (such as may be encountered in an extruder or similar processing device), the external applied heat necessary to melt the xylitol may be reduced significantly through the mechanical heat generated by the intense mixing action within the extruder. It is generally envisaged that the extruder will be heated to a temperature not less than 25°C below the melting point of the sugar alcohol, preferably in the range from 20°C below the melting point of the sugar alcohol to 50°C above the melting point of the drug, more preferably from 10°C below the melting point of the sugar alcohol to 30°C above its melting point and most preferably to a temperature in the range of 10°C to 30°C above the melting point of the sugar alcohol. Some extruders allow different zones to be heated to different temperatures in the extruder. These temperatures can be chosen as desired to ensure that the sugar alcohol is fully melted. Preferably, the sugar alcohol, paracetamol, NSAID salt and optional excipients, for example a disintegrant, are heated to a temperature in the range 80 to 180°C, more preferably 90 to 170°C, most preferably 110 to 150°C to melt said sugar alcohol. When the sugar alcohol is xylitol or D-sorbitol it may conveniently be heated in the range 100 to 160°C, more preferably 110-130°C. The sugar alcohol may also be heated and subjected to conditions of force, such as by heat-extruding the sugar alcohol, for example in a twin-screw extruder.

The sugar alcohol, paracetamol, NSAID salt and other optional excipients which may be present in the granules are preferably melted in a heated extruder barrel having an inlet for the solid mixture of the sugar alcohol and NSAID salt and an outlet for the molten extrudate. The barrel may be divided into different heating zones as desired. A suitable extruder arrangement is disclosed in International patent application PCT/GB02/02556.

The extruder may also have one or more cooling zones. The cooling zones may be necessary to remove the heat generated by the kneading action on the material being extruded, particularly to ensure that there is a good flow of material into the extruder and out from the extruder.

In a preferred process according to the present invention, the extruder is provided with a cooling zone and a heating zone. Further preferably, there is provided a cooling zone at the inlet portion of the extruder so that the material entering the extruder may be conveyed or transferred along the extruder to a heated zone. In the cooling zone, the internal heat generated within the material being extruded is carried away so that partial melting of the sugar alcohol cannot occur which may be detrimental to the throughput of material in the extruder. Preferably, the extruder is provided with a cooled transfer zone and a heated melting zone.

In a further preferred process, there is provided a heated zone at an end portion of the extruder at or adjacent the outlet. The extruded material may be heated to ensure that the extrudate passing through the extruder outlet is sufficiently heated so that the temperature difference between the molten extrudate and extrudate cooling means is maximised as appropriate to optimise the cooling process. For example, the barrel may be heated to cause the extrudate passing through the outlet to be preferably fully molten or substantially fully molten. The pressure within the extruder may cause a lowering of the melting point of the sugar alcohol. Accordingly, preferably, the temperature of the extrudate passing through the outlet is in the range of 20°C on each side of the normal melting point of the sugar alcohol, preferably within 10°C on each side of the melting point of the sugar alcohol.

The extruder is suitably provided with at least one screw shaft provided with means arranged to generate heat within the sugar alcohol. This may usually be achieved by a combination of kneading paddles and helical screws. Generally, it is preferred to provide helical screws at the inlet portion to convey the material away from the inlet. The material may be extruded in the extruder barrel with screws and/or with paddles. It is preferred to use more than one screw shaft, for example a twin-screw shaft, to maximise the extrusion effect on the material being extruded. The use of paddles also maximises the shear effect on the material being extruded. The paddles may be offset at any desired angle or combination of angles to generate internal heat within the sugar alcohol as appropriate to melt the sugar alcohol. The configuration and/or size of the paddles will depend on factors such as the diameter and/or length of the extruder, the ratio of the length to the diameter, the extruder speed, the torque applied and the desired temperature to melt the sugar alcohol. The screws and/or the paddles may be in the forward and/or reverse direction to maximise the pressure within the mixing zone as desired.

A preferred arrangement comprises helical transfer screws at inlet portion of the extruder, a plurality of paddles which may have differing sizes and degrees to which they are offset and further helical transfer screws at the outlet portion to convey the extrudate out of the extruder. Further preferably the helical transfer screws at the outlet portion may comprise a reverse helix followed by a forward helix.

When the sugar alcohol is substantially fully melted, a liquid is formed. The sugar alcohol should be fully melted so that on cooling, a single continuous amorphous phase of the sugar alcohol is formed. Suitably, the melting point of the sugar alcohol is substantially lower than the melting point of the NSAID salt. Thus the NSAID salt typically does not melt during the preparation of the pharmaceutical composition of the present invention. Preferably, the paracetamol does not melt during the preparation of the pharmaceutical composition. The NSAID salt and paracetamol may partially dissolve within the molten sugar alcohol and/or the NSAID salt and paracetamol may be dispersed within the molten sugar alcohol. Predominantly, the majority of the NSAID salt and paracetamol is uniformly dispersed within the molten sugar alcohol. Advantageously, the process of the present invention permits the formation of a pharmaceutical composition comprising a NSAID salt and paracetamol whilst minimising and/or preventing degradation of the NSAID salt and/or paracetamol. When used, additional components such as a disintegrant or an additional pharmaceutically active agent or any of the other excipients described above are combined with the sugar alcohol, paracetamol and NSAID salt, either prior to melting the sugar alcohol or after the melting process. The additional components used are often insoluble in the sugar alcohol, paracetamol and NSAID salt melt mixture and a dispersion of the additional components within the liquid melt is produced. The dispersion is mixed so that the additional component is uniformly or homogeneously combined with the melted sugar alcohol, paracetamol and NSAID salt mixture. A uniform mixture is thus produced. The mixture is allowed to cool by methods hereinafter discussed until a solid is produced. As the mixture cools, it becomes more viscous. The sugar alcohol which solidifies is then formed into melt granules. Thus, as used herein, "solidified melt granules" means granules formed from the sugar alcohol in molten form, preferably fully molten form, having the NSAID salt and paracetamol contained therein optionally with any additional component, cooling to a temperature below the melting point of the sugar alcohol and forming the solid mass into granules. The granular composition of the invention comprises a plurality of such granules.

The melt is allowed to solidify in any manner found convenient. This includes both rapid cooling and slow cooling. Preferably, the melt is cooled rapidly (i.e. quenched) thereby ensuring the solidified sugar alcohol forms a single continuous amorphous phase. For example, the molten mixture may be allowed to cool in a cooled vessel. The molten mixture may be poured onto cooling trays which may be static or continuously moving. Static trays may be placed in cooling cabinets. Moving trays or belts may have additional cooling means, such as cooled water. The cooled melt forms a solid and may be scraped off the belt or collected as it falls off one end of a continuously moving belt.

Preferably, the sugar alcohol is fully molten as it exits the extruder. The extrudate may consist of the molten sugar alcohol, paracetamol and NSAID salt contained therein, without additional ingredients, wherein the sugar alcohol is present as a single continuous amorphous phase and the NSAID salt and paracetamol is dissolved and/or dispersed therein. Optionally, the extrudate may contain additional components, for example one or more of a disintegrant, a surfactant and a diluent, which are blended within the molten sugar alcohol, paracetamol and NSAID salt.

Preferably, the extrudate is formed into two or more thin ribbons. This is preferably achieved by passing the molten extrudate through channels at the outlet which form streams or ribbons of extrudate which may be directed onto the cooling means, preferably a cooling belt or a cooling drum.

The ribbons of molten extrudate are cooled rapidly by said cooling means, i.e. the ribbons solidify in thin ribbons, which solidify in 5 minutes or less, preferably 1 minute or less (e.g. 0-60 seconds), more preferably in 50 seconds or less (e.g. 1-50 seconds), more preferably 1-40 seconds and most preferably 1-30 seconds.

Suitably, the width of each ribbon of molten extrudate is greater than the depth of the ribbon so that cooling is optimised. The width of each ribbon will, of course, depend, at least to some extent, on the viscosity of the molten material. Preferably, each ribbon of molten extrudate has a depth on the cooling means of up to thin ribbons, of 10 mm or less, preferably 0.1 to 6 mm, more preferably 0.5 to 5 mm, for example 3 to 4 mm and most preferably 1-3 mm, for example 2 mm.

Cooling will normally occur first on the side of the ribbon proximate the cooling means. Accordingly, usually the lower surface of the ribbons solidifies while the upper surface of the ribbon is still molten. As the ribbon is further cooled, the extrudate solidifies throughout its depth.

To maximise output, a plurality of ribbons are provided extending parallel to each other, for example on a cooling belt. Preferably, there are more than two ribbons, for example three, four, five, six, seven, eight, nine or ten or more ribbons according to the size of the extruder. The number of ribbons may be limited by the width of the ribbon formed and the whole width of the cooling means which provides for a maximum number of ribbons. It has been found that the ribbons of molten sugar alcohol, paracetamol and NSAID salt do not spread on the cooling means, accordingly there requires only a small space between the ribbons.

As hereinabove discussed, it is preferred to have a significant temperature difference between the molten extrudate as it comes into contact with the cooling means, for example at least 25°C, preferably at least 35°C, more preferably at least 45°C and most preferably at least 55°C. The upper end of the above ranges is limited by the melting point of the sugar alcohol, but it is not desired to heat the extruded material to too high a temperature as the extra energy costs will not be balanced by any processing advantage. Preferably, the molten extrudate is quenched by cooling it at a temperature of less than or equal to 30 °C, more preferably less than or equal to 20 °C, most preferably less than or equal to 15 °C.

Generally, it is expected that the molten mixture will be cooled to a temperature below the melting point of the sugar alcohol before being formed into granules. The molten mixture may be cooled by passing the molten mixture onto a moving cooling belt, preferably a continuously rotating cooling belt. Preferably, the belt is cooled by water. The water may be applied to the underside of the belt along its length or partially along its length as desired and according to the length of the belt, the quantity of molten mixture and the speed of the belt. It is especially preferred to cool the molten mixture at least initially by cooling means, for example until it has started to solidify. Advantageously, the belt is water-cooled along substantially the whole of its length and it is of minimum length required (e.g. 3-7m) to allow it to cool to the solid state.

The solidified melt may be formed into granules by a plurality of methods. For example, it may be pulverised into granules. It may be milled and/or sieved. It may also be passed through a spray device such as a spray tower or spray granulator in which the molten material is sprayed from an orifice into a stream of cooled air, allowed to congeal/solidify and then collected. If the molten sugar alcohol, paracetamol and NSAID salt mixture is extruded, the extrudate may be cooled and then broken into conveniently sized pieces, followed by milling and or sieving. Alternatively, the extrudate may be extruded through holes and chopped into suitably sized granules for tabletting. If it is cooled on a moving belt or drum, the cooled melt may be broken into conveniently sized pieces, followed by milling and or sieving.

The granular composition may be sieved to ensure that the melt granules are of the appropriate size for efficient tabletting. The granules produced on cooling the molten drug are preferably of a suitable size for tabletting, preferably in a standard large scale tabletting machine. The melt granules in the granular composition preferably have a mean particle size in the range 10-2000µm, more preferably 50-1000µm and most preferably 100-400µm. Valuable results are achieved when the bulk density of the melt granules is in the range 0.1-1gml⁻¹, more preferably 0.3-0.6gml⁻¹. The tapped density may be in the range 0.3-0.7gml⁻¹ (more preferably 0.4-0.6 gml⁻¹). The melt granules may have a porosity of 0.5-2.0 g/ml.

The sugar alcohol preferably forms a single continuous amorphous phase in the melt granule. That is to say substantially all of the sugar alcohol does not have a defined crystalline structure.

The solidified melt granules may be formulated directly or they may be combined with an extra-granular composition and formulated into a unit dose. Unexpectedly, the melt granules exhibit improved flow characteristics and are less flaky/sticky then the NSAID salt itself. Conveniently, the granules are typically easier to compress and do not stick to the punches of the tabletting machines. The melt granules may be combined thoroughly with extra-granular composition so as to form a uniform mixture of ingredients. This may be achieved by conventional mixing and blending techniques. Examples of apparatus that may be used to facilitate this process are: Ribbon Blender, IBC Blender, V-Blender and Plough Blenders. Examples include filling of the loose powder mixture into a sachet or a capsule or compressing it into a tablet. Tablets are the preferred unit dosage form according to the invention. They may be swallowed or they may be chewed. It has unexpectedly been found that the taste of the NSAID salt has been substantially masked which allows the dosage form to be maintained in the oral cavity for a period of time whilst the formulation is swallowed.

The compressed tablet composition of the present invention may optionally be coated with a film coat, for example based on a conventional cellulose polymer such as hydroxypropylmethylcellulose, or a conventional sugar coat, for example based on sucrose or lactose.

A preferred tablet composition according to the present invention may be prepared by incorporating silicon dioxide and optionally other excipients within the composition to be tabletted, preferably to form a powder blend, followed by compression into tablets.

In a preferred aspect of the present invention, there is provided a process to prepare a pharmaceutical composition as defined herein comprising the steps of:
a) forming a melt mixture comprising said molten sugar alcohol having said NSAID salt, paracetamol and a disintegrant uniformly contained therein; and
b) forming the melt mixture into solidified melt granules.

Preferably, the melt granules are mixed with extra-granular silicon dioxide to form a powder blend, followed by compression into tablets.

The invention is illustrated by the following non-limited examples. In the examples, sodium ibuprofen is available from Shasun Corporation, India or BASF, Germany; naproxen sodium is available from Divi's Laboratories, USA; sodium flurbiprofen dihydrate is available from DSM, USA; diclofenac monopotassium is available from Unique, India; paracetamol is available from Mallinckrodt, USA, croscarmellose sodium is available from FMC Corporation, Brussels, Belgium, under the tradename Ac-Di-Sol; D-sorbitol and xylitol are available from Roquette, France; French chalk is available from Luzenac, France; colloidal silicon dioxide (also known as colloidal silica) is available from Degussa, Frankfurt, Germany, under the tradename Aerosil 200; magnesium stearate is available from Hays Chemicals UK; stearic acid is available from Hays Chemicals UK; microcrystalline cellulose is available from the FMC Corporation, Brussels, Belgium, under the tradename Avicel PH101; dicalcium phosphate is available from Univar Limited UK under the tradename Emcompress; and Lactose NF Fast Flo is available from DMV in Holland.

### Crushing Strength

The crushing strength is a measure of the hardness of the tablet. It was measured by recording the diametrical crushing strength when the tablet was broken between the motorised jaws of a Schleuniger 6D Tablet Tester. The jaws of the tablet tester were set at a distance setting number of 24, 26, 28 and 29 on the instrument. The higher the distance setting number the greater the pressure applied to the tablet.

In Tables 1 and 2, the values in bold text indicate the components which form part of the melt granular composition and values in normal text indicate the components which form part of the extra-granular composition. The numerical values in these tables refer to the % by wt of each component present in the respective composition.

### Example 1(a): Preparation of the Granular Component

The process for all illustrative examples involves dry blending the sugar alcohol, paracetamol and NSAID salt, optionally with other excipients which may be present in the granular component, and then heating the mixture at a temperature of 100 to 165°C in an extruder to melt the sugar alcohol fully and thereby mix the molten sugar alcohol with non-molten NSAID salt, paracetamol and other optional excipients. The molten mass is poured onto cooled stainless steel trays or a cooled moving belt at 10 °C and allowed to cool. The molten mixture typically solidifies within 60 seconds; the mixture may be agitated during cooling. The solid mass thus formed is milled by passing through a cone mill having a screen with a round hole of 1mm. The resulting granules are collected.

### Example 1(b): Preparation of a tablet

The respective extra-granular components (shown in normal text in Tables 1 and 2), namely, colloidal silicon dioxide, magnesium stearate, stearic acid, lactose, dicalcium phosphate and microcrystalline cellulose are blended simultaneously with the granular composition formed from Example 1(a) above for approximately 15 minutes in a blender. The blended material was fed to a rotary tabletting machine (Fette P21 Hundred 2100) and compressed into tablets (machine speed of 150,000 tablets per hour and compaction force of from 4 kN to 14 kN) containing a therapeutic dose of NSAID drug and paracetamol.

### Examples 2 to 12

Tablets were prepared from the components in Table 1 in the same manner as described for Example 1. The compressing weight of each formulation is adjusted to give a tablet containing the desired therapeutic level of NSAID and paracetamol. In Examples 1 to 7 and 10, the sodium ibuprofen dihydrate is present in an amount of 256 mg per tablet. Consequently, the paracetamol is present in an amount of 325 mg per tablet for Examples 1, 3 and 10 and 500 mg per tablet for Examples 2, and 4 to 7. In Examples 8 and 9 the ibuprofen lysinate is present in an amount of 342 mg per tablet and the paracetamol present at 325 mg per tablet. However, it will be appreciated that a range of therapeutic doses of NSAID salt and paracetamol may be employed.

### Examples 11 so 18

Tablets were prepared from the components in Table 2 in the same manner as described in Example 1. The compressing weight of each formulation is adjusted to give a tablet containing the desired therapeutic level of NSAID. In this respect, naproxen sodium is present in an amount of 250 mg per tablet in Examples 11 to 14, diclofenac potassium salt is present in an amount of 75 mg per tablet in Examples 15 and 16, and flurbiprofen sodium is present in an amount of 75 mg per tablet in Examples 17 and 18. Suitably, the paracetamol is present in an amount of 317 mg per tablet in Examples 11 and 13, 488 mg per tablet in Examples 12 and 14, and 251 mg per tablet in Examples 15 to 18.

**Table 1**

| Formulation | % by wt present in formulation | | | | | | | | | |
|---|---|---|---|---|---|---|---|---|---|---|
| | 1 | 2 | 3 | 4 | 5 | 6 | 7 | 8 | 9 | 10 |
| xylitol | **4.8** | **6.9** | **4.8** | **6.9** | **6.3** | **6.3** | **6.8** | **6.2** | **6.2** | **4.8** |
| Sodium Ibuprofen | **37.6** | **28.2** | **37.6** | **28.2** | **26.8** | **26.8** | **28.3** | **-** | **-** | **37.6** |
| Paracetamol (Acetaminophen) | **47.7** | **55.1** | **47.7** | **55.1** | **52.4** | **52.4** | **55.2** | **40.3** | **40.3** | **47.7** |
| Ibuprofen Lysine | **-** | **-** | **-** | **-** | **-** | **-** | **-** | **42.4** | **42.4** | **-** |
| Croscarmellose sodium | **4.4** | **3.3** | **4.4** | **3.3** | **3.1** | **3.1** | **3.3** | **3.7** | **3.7** | **4.4** |
| Microcrystalline cellulose | 4.4 | 5.5 | - | - | 10.5 | - | 5.5 | 6.2 | - | - |
| Colloidal silicon dioxide | 0.4 | 0.3 | 0.4 | 0.3 | 0.3 | 0.3 | 0.3 | 0.4 | 0.4 | 0.4 |
| Magnesium stearate | 0.4 | 0.4 | 0.4 | 0.4 | 0.3 | 0.3 | 0.3 | 0.4 | 0.4 | 0.4 |
| Stearic acid | 0.3 | 0.3 | 0.3 | 0.3 | 0.3 | 0.3 | 0.3 | 0.4 | 0.4 | 0.3 |
| Lactose Fast Flo | - | - | 4.4 | 5.5 | - | 10.5 | - | - | 6.2 | - |
| Dicalcium phosphate | - | - | - | - | - | - | - | - | - | 4.4 |

| | | | | | | | | | | |
|---|---|---|---|---|---|---|---|---|---|---|
| * Note: Bold indicates the melt phase component | | | | | | | | | | |

**Table 2**

| Formulation | % by weight present in formulation | | | | | | | |
|---|---|---|---|---|---|---|---|---|
| | 11 | 12 | 13 | 14 | 15 | 16 | 17 | 18 |
| Naproxen Sodium^{*1} | **37.6** | **28.2** | **37.6** | **28.2** | - | - | **-** | **-** |
| Flurbiprofen Sodium^{*2} | **-** | **-** | **-** | **-** | **-** | **-** | **14.2** | **14.2** |
| Diclofenac Potassium salt^{*3} | **-** | **-** | **-** | **-** | **14.2** | **14.2** | **-** | **-** |
| Paracetamol | **47.7** | **55.1** | **47.7** | **55.1** | **47.7** | **47.7** | **47.7** | **47.7** |
| Croscarmellose Sodium | **4.4** | **3.3** | **4.4** | **3.3** | **8.3** | **8.3** | **8.3** | **8.3** |
| Sorbitol | **4.8** | **-** | **-** | **-** | **8.3** | **-** | **8.3** | **-** |
| Xylitol | **-** | **6.9** | **4.8** | **6.9** | **-** | **8.3** | **-** | **8.3** |
| Colloidal Silicon Dioxide | 0.4 | 0.3 | 0.4 | 0.3 | 1.0 | 1.0 | 1.0 | 1.0 |
| Magnesium Stearate | 0.4 | 0.4 | 0.4 | 0.4 | - | 1.0 | 1.0 | - |
| Stearic Acid | 0.3 | 0.3 | 0.3 | 0.3 | 1.0 | - | - | 1.0 |
| Microcrystalline Cellulose | 4.4 | 5.5 | - | - | 19.5 | - | 19.5 | - |
| Dixcalcium Phosphate | - | - | - | - | - | - | - | - |
| Lactose NF Fast Flo | - | - | 4.4 | 5.5 | - | 19.5 | - | 19.5 |

| | | | | | | | | |
|---|---|---|---|---|---|---|---|---|
| Note: Bold indicates melt phase component *1 6-Methoxy-alpha-methyl-2-naphthaleneacetic acid Sodium Salt *2 Sodium(+)-2-fluoro-a-methyl-4-biphenyl-acetate dihydrate *3 2-[(2,6-dichlorophenyl)amino] benzeneacetic acid, monopotassium salt | | | | | | | | |

### Example 19 - Improved flow characteristics and copressability of the pharmaceutical composition of the present invention compared with a dry blend formulation

Tablets comprising sodium ibuprofen and paracetamol were formed from the dry blend formulation as outlined below.

| | % by wt | mg/tablet |
|---|---|---|
| Sodium ibuprofen | 35.4 | 256 |
| Paracetamol | 45 | 325 |
| Xylitol | 4.1 | 30 |
| Anhydrous colloidal silicon dioxide | 0.3 | 2 |
| Magnesium stearate | 0.7 | 5 |
| Croscarmellose sodium | 4.1 | 30 |
| Lactose | 9.7 | 70 |
| Stearic acid | 0.7 | 5 |

The raw materials were sieved via a 16 mesh screen, apart from the anhydrous colloidal silicon dioxide which was passed through a 32 mesh screen, and dry blended together for 15 minutes on a tumble blender. The powder blend was then compressed on a Manesty F3 single punch tablet press using conventional 18 mm x 8 mm caplet tooling to a tablet weight of 723 mg. The mixture exhibited a sticky consistency and showed high levels of sticking to the tablet punches during compression.

In contrast, the formulation of Example 1 as detailed in Table 1 displayed excellent flow characteristics (i.e. non-sticky) and was easier to compress. It was possible to run the rotary tabletting machine at a fast rate of 150,000 tablets per hour.

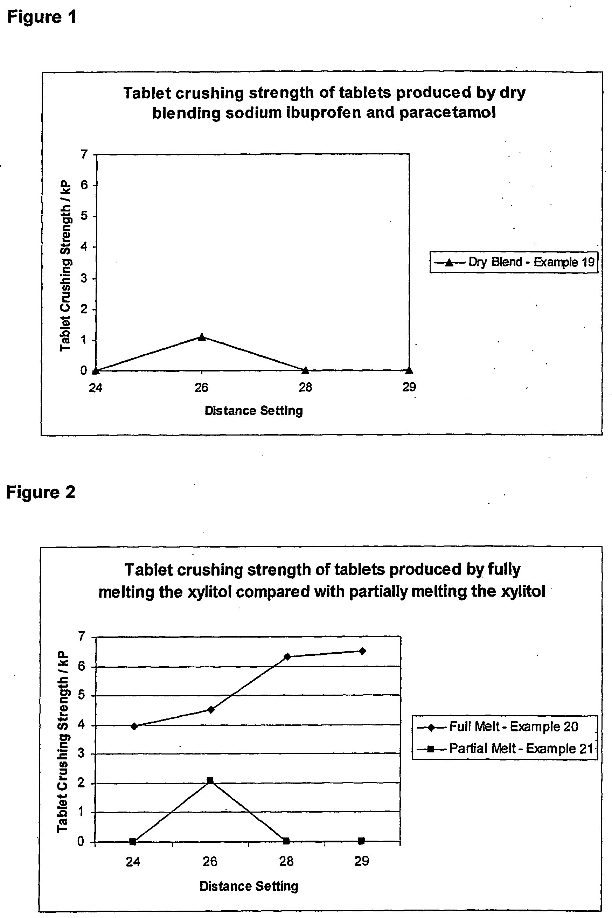

The tablet crushing strength of the tablets formed from the dry blend formulation was measured using a Schleuniger 6D Tablet Tester and the results are presented in Table 3 and Figure 1.

**Table 3**

| | **Tablet Crushing Strength / kP** | |
|---|---|---|
| **Distance Setting** | **Individual Results** | **Mean** |
| 24 | 0 | 0 |
| 26 | 1.2, 1.0, 0.9, 1.3, 1.1 | 1.01 |
| 28 | 0 | 0 |
| 29 | 0 | 0 |

The tablet crushing strength data confirms that tablets produced by a conventional dry blend process are extremely weak and brittle with a crushing strength around 1 kP at a distance setting of 26. Moreover, it was difficult to produce tablets by the dry blend process without lamination. Suitably, the tablets formed from the dry blend formulation are not particularly suited to withstand the further rigours of the manufacturing process (i.e. film or sugar coating packaging).

### Examples 20 and 21- Improved flow characteristics and compressibility of the pharmaceutical compositions of the present invention when the st/oa/' alcohol is fully melted compared to a comparable composition when the sugar alcohol is partially melted

The following tablets as detailed in Examples 20 and 21 below were prepared:

| Example | mg present in tablet | |
|---|---|---|
| | 20 | 21 |
| **Granular Component** | | |
| Sodium ibuprofen | 256 | 256 |
| Paracetamol | 325 | 325 |
| Xylitol | 30 | 30 |
| Croscarmellose sodium | 30 | 30 |
| **Extra Granular Component** | | |
| Lactose | 70 | 70 |
| Magnesium stearate | 5 | 5 |
| Stearic acid | 5 | 5 |
| Colloidal silicon dioxide | 2 | 2 |

The granular component of the tablets as detailed in Examples 20 and 21 were prepared as detailed in Example 1(a), except in Example 20 the xylitol was fully melted whereas in Example 21 the xylitol was partially melted.

The extra-granular components were sieved through a 16-mesh screen and blended with the respective granular components of Examples 20 and 21. The blended material was fed to a tabletting machine (Manesty F3 single punch tablets press using conventional 18 mm x 8 mm caplet tooling) and compressed into tablets. The tablet crushing strength of the tablets of Examples 20 and 21 was measured using a Schleunige 6D Tablet Tester and the results are presented in Table 4 and Figure 2.

**Table 4**

| | **Tablet Crushing Strength / kP** | | | |
|---|---|---|---|---|
| | **Example 20** | | **Example 21** | |
| **Distance Setting** | **Individual results** | **Mean Results** | **Individual Results** | **Mean Results** |
| 24 | 3.6, 3.9, 4.0, 4.3, 4.0 | 3.96 | 0 | 0 |
| 26 | 4.7, 4.5, 4.3, 4.4, 4.7 | 4.52 | 1.9, 2.0, 2.3, 2.1, 2.0 | 2.06 |
| 28 | 5.9, 6.1, 6.7, 6.4, 6.5 | 6.32 | 0 | 0 |
| 29 | 6.7, 6.5, 6.3, 6.5, 6.6 | 6.52 | 0 | 0 |

The extrudate produced by fully melting the xylitol (Example 20) was thin and pourable in consistency, whereas the extrudate produced by partially melting the xylitol although liquid was more viscous and had a paste like appearance. Both extrudates after cooling and milling produced melt granules having a similar visual appearance, but the melt granules produced by partially melting the xylitol (Example 21) were softer than granules produced by fully melting the xylitol (Example 20). These differences were also apparent when the granules were compressed into tablets - notably the granules formed by fully melting the xylitol (Example 20) exhibited improved flow characteristics and did not stick to the punches of the tabletting machine compared with the granules formed by partially melting the xylitol (Example 21). Suitably, the throughput and efficiency of the tabletting process may be increased by using granules where the xylitol is fully melted.

Moreover, as demonstrated by the results shown in Table 4 and Figure 2, the tablets formed from granules where the xylitol is fully melted (Example 20) are significantly more robust and harder than corresponding tablets where the xylitol is partially melted. Suitably, tablets formed by fully melting the xylitol will typically be able to withstand the further rigours of the manufacturing process (i.e. film coating and packaging) than tablets formed by partially melting the xylitol. I.

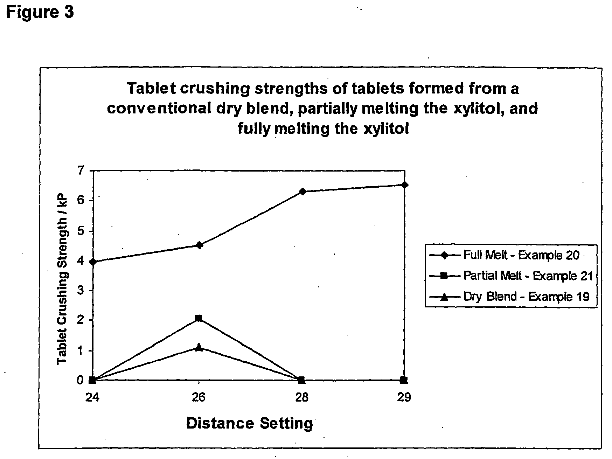

Figure 3 illustrates the differences in tablet crushing strength of sodium ibuprofen and paracetamol tablets produced from a dry blend formulation (Example 19), comparable tablets produced by partially melting the xylitol (Example 21) and comparable tablets produced by fully melting the xylitol (Example 20). Suitably, it is evident that tablets produced by fully melting the xylitol (Example 20) are more robust than tablets formed by partially melting the xylitol (Example 21), which in turn are more robust than tablets formed by a conventional dry blend process.

## Claims

1. A pharmaceutical composition comprising a granular component comprising a plurality of solidified melt granules of a sugar alcohol having a salt of a non-steroidal anti-inflammatory drug (NSAID salt) and paracetamol contained therein.

2. A pharmaceutical composition as claimed in claim 1 wherein the NSAID salt is uniformly dispersed within the sugar alcohol.

3. A pharmaceutical composition as claimed in claim 1 or 2 wherein the NSAID salt comprises a salt of ibuprofen, naproxen, flurbiprofen, fenoprofen, ketoprofen and fenbufen.

4. A pharmaceutical composition as claimed in claim 3 wherein the NSAID salt comprises a salt of ibuprofen and flurbiprofen.

5. A pharmaceutical composition as claimed in any one of the preceding claims wherein the NSAID salt comprises a salt of racemic ibuprofen.

6. A pharmaceutical composition as claimed in any one of the preceding claims wherein the NSAID salt is selected from an alkali metal salt, alkaline earth metal salt, amine salt or amino acid salt of the NSAID.

7. A pharmaceutical composition as claimed in claim 6 wherein the NSAID salt comprises an alkali metal salt of the NSAID.

8. A pharmaceutical composition as claimed in claim 7 wherein the NSAID salt comprises the potassium or sodium salt, especially the sodium salt.

9. A pharmaceutical composition as claimed in any one of the preceding claims wherein the solidified melt granules are obtainable by fully melting the sugar alcohol.

10. A pharmaceutical composition as claimed in any one of the preceding claims wherein the sugar alcohol is derivable from reducing a monosaccharide or a di-saccharide.

11. A pharmaceutical composition as claimed in any one of the preceding claims wherein the sugar alcohol has a melting point of between 80 °C to 170 °C, preferably a melting point of between 90 °C and 125 °C.

12. A pharmaceutical composition as claimed in any one of the preceding claims wherein the sugar alcohol comprises D-sorbitol, xylitol, adonitol, arabitol, meso-erythnitol or mixtures thereof.

13. A pharmaceutical composition as claimed in claim 12 wherein the sugar alcohol comprises D-sorbitol or xylitol, especially xylitol.

14. A pharmaceutical composition as claimed in any one of the preceding claims wherein the NSAID salt is present in an amount of greater than or equal to 20% by wt of the granular component.

15. A pharmaceutical composition as claimed in any one of the preceding claims wherein the sugar alcohol is present in an amount of less than or equal to 25% by wt of the granular component.

16. A pharmaceutical composition as claimed in any one of the preceding claims wherein the paracetamol is uniformly dispersed in the sugar alcohol.

17. A pharmaceutical composition as claimed in any one of the preceding claims wherein the paracetamol is present in an amount of greater than or equal to 30% by wt of the granular component.

18. A pharmaceutical composition as claimed in any one of the preceding claims wherein the composition further includes a disintegrant.

19. A pharmaceutical composition as claimed in claim 18 wherein the disintegrant is present within the granular component of the pharmaceutical composition.

20. A pharmaceutical composition as claimed in any one of the preceding claims wherein the composition additionally includes at least one further pharmaceutically active ingredient.

21. A pharmaceutical composition as claimed in any one of the preceding claims wherein the formulation further includes a wicking agent that is insoluble in water.

22. A pharmaceutical composition as claimed in claim 21 wherein the wicking agent is silicon dioxide.

23. A pharmaceutical composition as claimed in claim 21 or 22 wherein the wicking agent is present in an amount of 0.1 to 5% by wt of the pharmaceutical composition.

24. A pharmaceutical composition as claimed in any one of claims 21 to 23 wherein the wicking agent is present within an extra-granular component of the composition.

25. A pharmaceutical composition as claimed in any one of the preceding claims wherein the composition further includes a surfactant.

26. A pharmaceutical composition as claimed in any one of the preceding claims wherein the composition further includes a diluent.

27. A pharmaceutical composition as claimed in any one of the preceding claims wherein the composition includes an extra-granular component.

28. A pharmaceutical composition as claimed in claim 27 wherein the extra-granular component includes a lubricant.

29. A pharmaceutical composition as claimed in any one of the preceding claims wherein the formulation is in the form of an effervescent formulation, a chewable tablet, a powder mixture or a non-effervescent compressed tablet.

30. A pharmaceutical composition as claimed in any one of the preceding claims wherein the formulation is in the form of a non-effervescent compressed tablet.

31. A pharmaceutical composition as claimed in any one of the preceding claims for use in the treatment of pain and/or inflammation and/or fever.

32. A pharmaceutical composition as claimed in claim 31 for use in the treatment of coughs, colds, influenza, migraine, headache, rheumatic pain, arthritic pain, muscular pain and/or neuralgia.

33. The use of a pharmaceutical composition as claimed in any one of claims 1 to 30 for the preparation of a medicament for use in the treatment of pain and/or inflammation and/or fever.

34. A process for producing a formulation according to any one of claims 1 to 32, which process comprises the steps of:
(a) forming a melt mixture comprising said molten sugar alcohol incorporating said NSAID salt and paracetamol therein, optionally said melt containing one or more additional excipients that may be present in the granules;
(b) forming the melt mixture into solidified melt granules.

35. A process as claimed in claim 34 wherein the sugar alcohol is fully molten in the melt mixture.

36. A process as claimed in claim 34 or 35 wherein the melt mixture is formed by mixing the NSAID salt and paracetamol with the sugar alcohol and then melting the sugar alcohol.

37. A process as claimed in claim 34 or 35 wherein the melt mixture is formed by melting the sugar alcohol and then adding the NSAID salt and paracetamol to the molten sugar alcohol.

38. A process as claimed in any one of claims 34 to 37 wherein the melt mixture is formed into solidified melt granules by cooling the melt mixture to form a solidified melt and comminuting the solidified melt.

39. A process as claimed in any one of claims 34 to 38 further including the step of compressing said solidified melt granules, optionally with an extra-granular component, to form a compressed tablet composition.

40. Use of a water insoluble wicking agent as an extra-granular component combined with a granular component in a compressed composition for enhancing the dispersion of the compressed composition in aqueous conditions, wherein the granular component comprises a plurality of solidified melt granules of a sugar alcohol incorporating a NSAID salt and paracetamol therein.

## Patentansprüche

1. Pharmazeutische Zusammensetzung, umfassend eine granulöse Komponente umfassend eine Mehrzahl erstarrter Schmelzgranulatteilchen eines Zuckeralkohols mit einem darin enthaltenen Salz eines nichtsteroidalen entzündungshemmenden Arzneimittels (NSAID-Salz, NSAID = Non-Steroidal Anti-Inflammatory Drug) und darin enthaltenem Paracetamol.

2. Pharmazeutische Zusammensetzung nach Anspruch 1, wobei das NSAID-Salz gleichmäßig im Zuckeralkohol verteilt ist.

3. Pharmazeutische Zusammensetzung nach Anspruch 1 oder 2, wobei das NSAID-Salz ein Salz von Ibuprofen, Naproxen, Flurbiprofen, Fenoprofen, Ketoprofen und Fenbufen umfasst.

4. Pharmazeutische Zusammensetzung nach Anspruch 3, wobei das NSAID-Salz ein Salz von Ibuprofen und Flurbiprofen umfasst.

5. Pharmazeutische Zusammensetzung nach einem der vorhergehenden Ansprüche, wobei das NSAID-Salz ein Salz von racemischem Ibuprofen umfasst.

6. Pharmazeutische Zusammensetzung nach einem der vorhergehenden Ansprüche, wobei das NSAID-Salz aus einem Alkalisalz, Erdalkalisalz, Aminsalz oder Aminosäuresalz des NSAID ausgewählt ist.

7. Pharmazeutische Zusammensetzung nach Anspruch 6, wobei das NSAID-Salz ein Alkalisalz des NSAID umfasst.

8. Pharmazeutische Zusammensetzung nach Anspruch 7, wobei das NSAID-Salz das Kalium- oder Natriumsalz, insbesondere das Natriumsalz, umfasst.

9. Pharmazeutische Zusammensetzung nach einem der vorhergehenden Ansprüche, wobei die erstarrten Schmelzgranulatteilchen durch vollständiges Aufschmelzen des Zuckeralkohols erhältlich sind.

10. Pharmazeutische Zusammensetzung nach einem der vorhergehenden Ansprüche, wobei der Zuckeralkohol durch Reduzieren eines Monosaccharids oder eines Disaccharids erhältlich ist.

11. Pharmazeutische Zusammensetzung nach einem der vorhergehenden Ansprüche, wobei der Zuckeralkohol einen Schmelzpunkt von 80°C bis 170°C, vorzugsweise einen Schmelzpunkt von 90°C bis 125°C aufweist.

12. Pharmazeutische Zusammensetzung nach einem der vorhergehenden Ansprüche, wobei der Zuckeralkohol D-Sorbit, Xylit, Adonit, Arabit, meso-Erythnit oder Mischungen davon umfasst.

13. Pharmazeutische Zusammensetzung nach Anspruch 12, wobei der Zuckeralkohol D-Sorbit oder Xylit, insbesondere Xylit, umfasst.

14. Pharmazeutische Zusammensetzung nach einem der vorhergehenden Ansprüche, wobei das NSAID-Salz in einer Menge von größer als oder gleich 20 Gew.-% der granulösen Komponente vorliegt.

15. Pharmazeutische Zusammensetzung nach einem der vorhergehenden Ansprüche, wobei der Zuckeralkohol in einer Menge von weniger als oder gleich 25 Gew.-% der granulösen Komponente vorliegt.

16. Pharmazeutische Zusammensetzung nach einem der vorhergehenden Ansprüche, wobei das Paracetamol gleichmäßig im Zuckeralkohol verteilt ist.

17. Pharmazeutische Zusammensetzung nach einem der vorhergehenden Ansprüche, wobei das Paracetamol in einer Menge von größer als oder gleich 30 Gew.-% der granulösen Komponente vorliegt.

18. Pharmazeutische Zusammensetzung nach einem der vorhergehenden Ansprüche, wobei die Zusammensetzung weiterhin ein Sprengmittel einschließt.

19. Pharmazeutische Zusammensetzung nach Anspruch 18, wobei das Sprengmittel in der granulösen Komponente der pharmazeutischen Zusammensetzung vorliegt.

20. Pharmazeutische Zusammensetzung nach einem der vorhergehenden Ansprüche, wobei die Zusammensetzung zusätzlich mindestens einen weiteren pharmazeutischen Wirkstoff einschließt.

21. Pharmazeutische Zusammensetzung nach einem der vorhergehenden Ansprüche, wobei die Formulierung weiterhin ein in Wasser unlösliches, als Docht wirkendes Mittel einschließt.

22. Pharmazeutische Zusammensetzung nach Anspruch 21, wobei es sich bei dem als Docht wirkenden Mittel um Siliciumdioxid handelt.

23. Pharmazeutische Zusammensetzung nach Anspruch 21 oder 22, wobei das als Docht wirkende Mittel in einer Menge von 0,1 bis 5 Gew.-% der pharmazeutischen Zusammensetzung vorliegt.

24. Pharmazeutische Zusammensetzung nach einem der Ansprüche 21 bis 23, wobei das als Docht wirkende Mittel in einer extragranulösen Komponente der Zusammensetzung vorliegt.

25. Pharmazeutische Zusammensetzung nach einem der vorhergehenden Ansprüche, wobei die Zusammensetzung weiterhin ein Tensid einschließt.

26. Pharmazeutische Zusammensetzung nach einem der vorhergehenden Ansprüche, wobei die Zusammensetzung weiterhin ein Verdünnungsmittel einschließt.

27. Pharmazeutische Zusammensetzung nach einem der vorhergehenden Ansprüche, wobei die Zusammensetzung eine extragranulöse Komponente einschließt.

28. Pharmazeutische Zusammensetzung nach Anspruch 27, wobei die extragranulöse Komponente ein Schmiermittel einschließt.

29. Pharmazeutische Zusammensetzung nach einem der vorhergehenden Ansprüche, wobei die Formulierung in Form einer Brauseformulierung, einer Kautablette, einer Pulvermischung oder einer nichtaufbrausenden komprimierten Tablette vorliegt.

30. Pharmazeutische Zusammensetzung nach einem der vorhergehenden Ansprüche, wobei die Formulierung in Form einer nichtaufbrausenden komprimierten Tablette vorliegt.

31. Pharmazeutische Zusammensetzung nach einem der vorhergehenden Ansprüche zur Verwendung bei der Behandlung von Schmerzen und/oder Entzündung und/oder Fieber.

32. Pharmazeutische Zusammensetzung nach Anspruch 31 zur Verwendung bei der Behandlung von Husten, Erkältungen, Grippe, Migräne, Kopfschmerzen, rheumatischen Schmerzen, arthritischen Schmerzen, Muskelschmerzen und/oder Neuralgie.

33. Verwendung einer pharmazeutischen Zusammensetzung nach einem der Ansprüche 1 bis 30 zur Herstellung eines Medikaments zur Verwendung bei der Behandlung von Schmerzen und/oder Entzündung und/oder Fieber.

34. Verfahren zur Herstellung einer Formulierung nach einem der Ansprüche 1 bis 32, welches die folgenden Schritte umfasst:
(a)Bilden einer den geschmolzenen Zuckeralkohol umfassenden Schmelzmischung unter Einarbeitung des NSAID-Salzes und des Paracetamols, wobei die Schmelze gegebenenfalls einen oder mehrere zusätzliche Exzipienten enthält, die in den Granulatteilchen vorliegen können,
(b)Formen der Schmelzmischung zu erstarrten Schmelzgranulatteilchen.

35. Verfahren nach Anspruch 34, wobei der Zuckeralkohol in der Schmelzmischung vollständig geschmolzen vorliegt.

36. Verfahren nach Anspruch 34 oder 35, bei dem man die Schmelzmischung durch Mischung des NSAID-Salzes und des Paracetamols mit dem Zuckeralkohol und anschließendem Schmelzen des Zuckeralkohols bildet.

37. Verfahren nach Anspruch 34 oder 35, bei dem man die Schmelzmischung durch Schmelzen des Zuckeralkohols und anschließende Zugabe des NSAID-Salzes und des Paracetamols zum geschmolzenen Zuckeralkohol bildet.

38. Verfahren nach einem der Ansprüche 34 bis 37, bei dem man die Schmelzmischung zu erstarrten Schmelzgranulatteilchen formt, indem man die Schmelzmischung unter Bildung einer erstarrten Schmelze abkühlt und die erstarrte Schmelze zerkleinert.

39. Verfahren nach einem der Ansprüche 34 bis 38, welches weiterhin den Schritt des Komprimierens der erstarrten Schmelzgranulatteilchen, gegebenenfalls mit einer extragranulösen Komponente, unter Bildung einer komprimierten Tablettenzusammensetzung umfasst.

40. Verwendung eines wasserunlöslichen, als Docht wirkenden Mittels als extragranulöse Komponente kombiniert mit einer granulösen Komponente in einer komprimierten Zusammensetzung zur Verbesserung der Verteilung der komprimierten Zusammensetzung unter wässrigen Bedingungen, wobei die granulöse Komponente eine Mehrzahl erstarrter Schmelzgranulatteilchen eines Zuckeralkohols mit darin eingearbeitetem NSAID-Salz und Paracetamol umfasst.

## Revendications

1. Composition pharmaceutique comprenant un composant granulaire comprenant une multitude de granules fondus solidifiés d'un alcool sucré contenant un sel d'un anti-inflammatoire non stéroïdien (sel d'AINS) et du paracétamol.

2. Composition pharmaceutique selon la revendication 1, où le sel d'AINS est dispersé uniformément à l'intérieur de l'alcool sucré.

3. Composition pharmaceutique selon la revendication 1 ou 2, où le sel d'AINS comprend un sel d'ibuprofène, de naproxène, de flurbiprofène, de fénoprofène, de kétoprofène et de fenbufène.

4. Composition pharmaceutique selon la revendication 3, où le sel d'AINS comprend un sel d'ibuprofène et de flurbiprofène.

5. Composition pharmaceutique selon l'une quelconque des revendications précédentes, où le sel d'AINS comprend un sel d'ibuprofène racémique.

6. Composition pharmaceutique selon l'une quelconque des revendications précédentes, où le sel d'AINS est choisi parmi un sel de métal alcalin, un sel de métal alcalino-terreux, un sel d'amine ou un sel d'acide aminé de l'AINS.

7. Composition pharmaceutique selon la revendication 6, où le sel d'AINS comprend un sel de métal alcalin de l'AINS.

8. Composition pharmaceutique selon la revendication 7, où le sel d'AINS comprend le sel de potassium ou de sodium, spécialement le sel de sodium.

9. Composition pharmaceutique selon l'une quelconque des revendications précédentes, où les granules fondus solidifiés peuvent être obtenus par fusion complète de l'alcool sucré.

10. Composition pharmaceutique selon l'une quelconque des revendications précédentes, où l'alcool sucré peut être dérivé par la réduction d'un monosaccharide ou d'un disaccharide.

11. Composition pharmaceutique selon l'une quelconque des revendications précédentes, où l'alcool sucré présente un point de fusion compris entre 80 °C et 170 °C, préférentiellement un point de fusion compris entre 90 °C et 125 °C.

12. Composition pharmaceutique selon l'une quelconque des revendications précédentes, où l'alcool sucré comprend le D-sorbitol, le xylitol, l'adonitol, l'arabitol, le méso-érythnitol ou leurs mélanges.

13. Composition pharmaceutique selon la revendication 12, où l'alcool sucré comprend le D-sorbitol ou le xylitol, spécialement le xylitol.

14. Composition pharmaceutique selon l'une quelconque des revendications précédentes, où le sel d'AINS est présent à une teneur supérieure ou égale à 20 % en masse du composant granulaire.

15. Composition pharmaceutique selon l'une quelconque des revendications précédentes, où l'alcool sucré est présent à une teneur inférieure ou égale à 25 % en masse du composant granulaire.

16. Composition pharmaceutique selon l'une quelconque des revendications précédentes, où le paracétamol est dispersé de façon uniforme dans l'alcool sucré.

17. Composition pharmaceutique selon l'une quelconque des revendications précédentes, où le paracétamol est présent à une teneur supérieure ou égale à 30 % en masse du composant granulaire.

18. Composition pharmaceutique selon l'une quelconque des revendications précédentes, où la composition inclut en outre un agent délitant.

19. Composition pharmaceutique selon la revendication 18, où l'agent délitant est présent à l'intérieur du composant granulaire de la composition pharmaceutique.

20. Composition pharmaceutique selon l'une quelconque des revendications précédentes, où la composition inclut en outre au moins un principe actif pharmaceutique supplémentaire.

21. Composition pharmaceutique selon l'une quelconque des revendications précédentes, où la formule inclut en outre un agent absorbant qui est insoluble dans l'eau.

22. Composition pharmaceutique selon la revendication 21, où l'agent absorbant est le dioxyde de silicium.

23. Composition pharmaceutique selon la revendication 21 ou 22, où l'agent absorbant est présent à une teneur comprise entre 0,1 et 5 % en masse de la composition pharmaceutique.

24. Composition pharmaceutique selon l'une quelconque des revendications 21 à 23, où l'agent absorbant est présent à l'intérieur d'un composant extra-granulaire de la composition.

25. Composition pharmaceutique selon l'une quelconque des revendications précédentes, où la composition inclut en outre un tensioactif.

26. Composition pharmaceutique selon l'une quelconque des revendications précédentes, où la composition inclut en outre un diluant.

27. Composition pharmaceutique selon l'une quelconque des revendications précédentes, où la composition inclut en outre un composant extra-granulaire.

28. Composition pharmaceutique selon la revendication 27, où le composant extra-granulaire inclut un lubrifiant.

29. Composition pharmaceutique selon l'une quelconque des revendications précédentes, où la formule se présente sous la forme d'une formule effervescente, d'un comprimé à mâcher, d'un mélange en poudre ou d'un comprimé compressé non effervescent.

30. Composition pharmaceutique selon l'une quelconque des revendications précédentes, où la formule se présente sous la forme d'un comprimé compressé non effervescent.

31. Composition pharmaceutique selon l'une quelconque des revendications précédentes, pour utilisation dans le traitement de la douleur et/ou de l'inflammation et/ou de la fièvre.

32. Composition pharmaceutique selon la revendication 31 pour utilisation dans le traitement des toux, rhumes, grippe, migraines, céphalées, douleur rhumatismale, douleur arthritique, douleur musculaire et/ou névralgie.

33. Utilisation d'une composition pharmaceutique selon l'une quelconque des revendications 1 à 30 dans l'élaboration d'un médicament pour utilisation dans le traitement de la douleur et/ou de l'inflammation et/ou de la fièvre.

34. Procédé de production d'une formule selon l'une quelconque des revendications 1 à 32, ledit procédé comprenant les étapes suivantes :
(a) formation d'un mélange fondu comprenant ledit alcool sucre fondu incorporant en son sein ledit sel d'AINS et le paracétamol, ledit mélange fondu contenant éventuellement un ou plusieurs excipients supplémentaires pouvant être présents dans les granules ;
(b) façonnage du mélange fondu en granules fondus solidifiés.

35. Procédé selon la revendication 34, où l'alcool sucré est entièrement fondu dans le mélange fondu.

36. Procédé selon la revendication 34 ou 35, où le mélange fondu est formé par mélangeage du sel d'AINS et du paracétamol avec l'alcool sucré puis fusion de l'alcool sucré.

37. Procédé selon la revendication 34 ou 35, où le mélange fondu est formé par fusion de l'alcool sucré puis ajout du sel d'AINS et du paracétamol à l'alcool sucré fondu.

38. Procédé selon l'une quelconque des revendications 34 à 37, où le mélange fondu est façonné en granules fondus solidifiés par refroidissement du mélange fondu pour former un mélange fondu solidifié et broyage du mélange fondu solidifié.

39. Procédé selon l'une quelconque des revendications 34 à 38, incluant en outre l'étape de compression desdits granules fondus solidifiés, éventuellement avec un composant extra-granulaire, pour former une composition de comprimé compressé.

40. Utilisation d'un agent absorbant insoluble dans l'eau en tant que composant extra-granulaire combiné à un composant granulaire dans une composition compressée pour améliorer la dispersion de la composition compressée dans des conditions aqueuses, où le composant granulaire comprend une multitude de granules fondus solidifiés d'un alcool sucré incorporant un sel d'AINS et du paracétamol.
